# EUROPEAN PATENT SPECIFICATION

(11) **EP 3 228 302 B1**
(45) Date of publication and mention of the grant of the patent: **02.09.2020**
(21) Application number: 15865763.5
(22) Date of filing: 17.11.2015
(51) Int. Cl.: A61K 8/365, A61K 8/34, A61Q 5/04

(54) **HAIR DEFORMATION TREATMENT AGENT**
BEHANDLUNGSMITTEL ZUR HAARVERFORMUNG
AGENT DE TRAITEMENT DE DÉFORMATION DES CHEVEUX

(30) Priority: 05.12.2014 JP 2014247485
(43) Date of publication of application: 11.10.2017
(73) Proprietor: Kao Corporation, Chuo-ku Tokyo 103-8210 (JP)
(72) Inventor: FURUKAWA, Junichi, Tokyo 131-8501 (JP); TOKUNAGA, Shinichi, Tokyo 131-8501 (JP); TSUCHIYA, Masaru, Haga-gun Tochigi 321-3497 (JP)
(74) Representative: Hoffmann Eitle
(86) International application number: PCT/JP2015/082284
(87) International publication number: WO 2016/088551

(56) References cited:
- WO-A2-2012/010351
- WO-A2-2014/068101
- JP-A- 2005 194 261
- JP-A- 2005 194 261
- JP-A- 2013 520 468
- JP-A- 2013 531 046
- US-A- 3 910 289
- US-A1- 2006 096 042
- BOGA, C. ET AL.: 'Formaldehyde replacement with glyoxylic acid in semipermanent hair straightening: a new and multidisciplinary investigation' INTERNATIONAL JOURNAL OF COSMETIC SCIENCE vol. 36, no. 5, 11 October 2014, pages 459 - 470, XP055173854 DOI: 10.1111/ICS.12148

## Description

### [Field of the Invention]

The present invention relates to an agent for hair deforming treatment capable of semi-permanently or permanently deforming the shape of hair, the invention in the following being strictly limited to the content of the appended claims.

### [Background of the Invention]

As methods for semi-permanently or permanently deforming the shape of hair, there have been a method of using a reducing agent, as in the case of the formation of a permanent wave, and a method of using a strongly-alkaline treatment agent having a pH of from 12 to 14, including an alkali relaxer as a representative example. However, it has been well known that these methods pose a great burden on hair, and damage the hair. In addition, in recent years, as a method for semi-permanently or permanently the shape of hair with less damage to the hair, a hair-relaxing method, in which a large amount of formaldehyde is used, has been developed. However, such a method of using highly toxic formaldehyde must be handled carefully, because of its high volatility, and thus, it cannot be said that this is a preferred method of hair treatment.

Hence, a hair-relaxing method, which does not impart damage to hair and does not use formaldehyde, and which is safer to a human body, has been searched. For example, Patent Document 1 discloses a technology of applying α-keto acid, and particularly, glyoxylic acid to hair, and then subjecting the hair to a heat treatment with a flat iron at 200°C ± 50°C, to convert very curly hair to a straight hair. Moreover, Patent Document 2 discloses a method which comprises applying a polyhydroxylated aromatic compound to hair, and then heating the hair to a temperature of 110°C or higher, to permanently relax keratin fibers.

With regard to temporary hair deformation, such as the resetting of a hair style by rinsing the hair with water, it can be realized by using a hair styling agent. For example, Patent Document 3 discloses a hair cosmetic composition, which is prepared by heating glyceraldehyde and resorcin to reflux in the presence of boric acid and silicic acid, to form an oligomer. Patent Document 3 describes that this composition improves set retentivity and humidity resistance, is capable of reforming the hair style by wetting the hair with water, and improves the mechanical strength of hair.

[Patent Document 1] EP 2538916 A
[Patent Document 2] JP-A-2009-537619
[Patent Document 3] US 4278659 B

US 2006/096042 is directed to a process for simultaneously coloring and permanently restructuring the hair, wherein (a) an oxidative hair colorant is applied to the hair based on at least one developer substance and least one coupler substance, (b) the oxidative hair colorant is left on for 5 to 60 minutes, (c) the oxidative hair colorant is rinsed out of the hair, (d) an acidic intermediate treatment agent with a pH of from 2 to 6 is applied to the hair, (e) after an action period of from 1 to 10 minutes, the intermediate treatment agent is rinsed out, if necessary, and the hair is then rolled up onto curlers, (f) a keratin-reducing permanent restructuring agent is applied to the hair, (g) after an action period of from 1 to 30 minutes, the keratin-reducing agent is rinsed out, if necessary, (h) the hair is fixed with an oxidative material, (i) after an action period of from 3 to 15 minutes, the hair is rinsed with water, if necessary and then treated with an acidic rinse.
US 3,910,289 describes a hair shaping composition comprising a mercapto salt of a carboxylic acid together with a salt of ascorbic or glyoxylic acid. The permanency of the shaping and elasticity of the hair are improved.
WO 2012/010351 relates to a treatment of human hair by means of solutions of glyoxylic acid that, when used in combination with mechanical straightening by means of hair-straigthening irons set at a temperature of approximately 200 °C +/- 30 °C, allow for semipermanently changing the shape of hair from curly and/or frizzy to straight for at least six consecutive washings with water and shampoo. JP 2005/194261 describes a frizzled hair-correcting agent capable of correcting the frizzled hair such as naturally kinky hair, without damaging and also excellent in its lasting effect. The agent comprises a specific substituted benzoic acid compound, furan carboxylic acid compound or alpha-ketocarboxylic acid compound.

### [Summary of the Invention]

The present invention provides an agent for hair deforming treatment comprising the following components (A), (B) and (C):
(A) glyoxylic acid or a hydrate or salt thereof,
B) a compound represented by the following formula (1), which has a molecular weight of more than 120, or a compound represented by the following formula (1-3-a) or (1-3-b) and
(C) water:

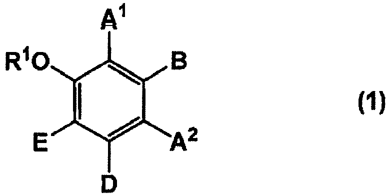

wherein, in the formula (1), R1 represents a hydrogen atom or a methyl group, A1 and A2, which are optionally the same or different, each represent a hydrogen atom, a straight-chain or branched-chain alkyl group or alkenyl group containing from 1 to 12 carbon atoms, an optionally substituted aralkyl group or arylalkenyl group containing from 7 to 12 carbon atoms, a straight-chain or branched-chain alkoxy group or alkenyloxy group containing from 1 to 6 carbon atoms, a halogen atom, or -CO-R2 (wherein R2 represents a straight-chain or branched-chain alkyl group or alkenyl group containing from 1 to 12 carbon atoms, an optionally substituted aralkyl group or arylalkenyl group containing from 7 to 12 carbon atoms, or an optionally substituted aromatic hydrocarbon group containing from 6 to 12 carbon atoms), B represents a hydrogen atom, a straight-chain or branched-chain alkyl group or alkenyl group containing from 1 to 12 carbon atoms, an optionally substituted aralkyl group or arylalkenyl group containing from 7 to 12 carbon atoms, - OR3 , or -COOR3 (wherein R3 represents a hydrogen atom, or a straight-chain or branched-chain alkyl group or alkenyl group containing from 1 to 6 carbon atoms), D represents a hydroxy group or a straight-chain or branched-chain alkoxy group or alkenyloxy group containing from 1 to 12 carbon atoms, and E represents a hydrogen atom, a hydroxy group, a straight-chain or branched-chain alkyl group or alkenyl group containing from 1 to 6 carbon atoms, or a straight-chain or branched-chain alkoxy group or alkenyloxy group containing from 1 to 6 carbon atoms, provided that two or three of A1 , A2, B and E are hydrogen atoms, and the remaining groups do not include sulfo groups;
from 1 to 6 carbon atoms, or a straight-chain or branched-chain alkoxy group or alkenyloxy group containing from 1 to 6 carbon atoms,
provided that two or three of A¹, A², B and E are hydrogen atoms, and the remaining groups do not include sulfo groups, and that when D is a hydrogen atom or a methyl group, A¹ and B, or A² and B, together with two carbon atoms adjacent to them, form a benzene ring optionally substituted with a hydroxy group, and 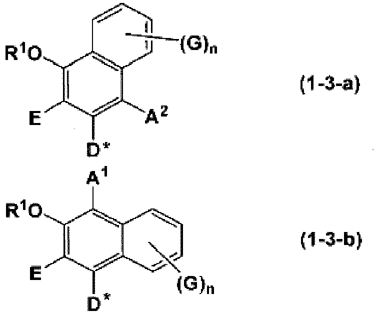 wherein, in the formulae (1-3-a) and (1-3-b),
   R¹, A¹, A² and E are as defined above;
   D* represents a hydrogen atom, hydroxy group, a methyl group or a straight-chain or branched-chain alkoxy group or alkenyloxy group containing from 1 to 12 carbon atoms;
   G represents a hydroxy group, a straight-chain or branched-chain alkyl group or alkenyl group containing from 1 to 6 carbon atoms, or an alkoxy group containing from 1 to 6 carbon atoms, and
   n represents an integer of from 0 to 2.

When the hair treatment agent used in the method described in Patent Document 1 or 2 is applied to hair, although it could convert the hair to straight hair semi-permanently, it could not provide a semi-permanently or permanently wavy or curly shape to the hair. In addition, when the once formed semi-permanent or permanent straight shape intends to be converted to another semi-permanent or permanent hair shape such as a wavy or curly shape, it is necessary to perform again the conventional operation of using a reducing agent. Thus, excessive amounts of time and efforts are required, and further, hair may be damaged.

On the other hand, the technology described in Patent Document 3 is characterized in that it is washed away with water because an oligomer is used as a treatment agent. As such, because the hair is returned to its original hair shape by being repeatedly washed, this technology cannot be considered to be semi-permanent or permanent hair deformation.

Accordingly, the present invention relates to an agent for hair deforming treatment and a method of hair treatment, which are safe for human bodies and have less damage to hair, are able to semi-permanently or permanently provide not only the shape of straight hair, but also a wavy or curly shape, and further, are able to easily convert the once formed hair style to another semi-permanent or permanent hair shape, without using an agent for hair deforming treatments such as a reducing agent, and without damaging to the hair.

The present inventors have found that a hair treatment agent comprising glyoxylic acid and a specific resorcin analog cannot only semi-permanently provide a straight shape or a curly or wavy shape to hair, but also deform hair, which has once been treated with this hair treatment agent, into any completely different, any hair shape, only by using a heating means such as a hair iron or a curler, without treating the hair with a hair treatment agent such as a reducing agent, thereby completing the present invention.

The agent for hair deforming treatment of the present invention is highly safe for human bodies, has less damage to hair, and can deform the shape of hair semi-permanently or permanently, and the hair deformed with the present agent for hair deforming treatment does not lose its effect even if the hair is washed with a shampoo or the like. In addition, once the present agent for hair deforming treatment is applied to hair, it is not necessary to allow a hair treatment agent to penetrate into the hair again, and the hair can be freely and repeatedly deformed, semi-permanently or permanently, only by heating to the hair. Moreover, the hair, which has been arbitrarily repeatedly deformed by being heated, still has a high hair washing resistance of the shape of the hair, and thus the shape of the hair does not lose its effect by shampoo, water, etc.

In the present invention, "semi-permanent or permanent hair deformation" means that hair has extremely excellent hair washing resistance, and that the shape of the hair does not change, even if shampooing is repeatedly carried out thereon. Specifically, it means that, when the deformed hair is washed with shampoo, and the shampoo is then fully washed away with water, and the hair is then naturally dried, the shape of hair is maintained before and after shampooing. It is to be noted that the expression "the shape of hair is maintained" means that, for example, in the case of wavy hair, the number of waves is not substantially different before and after shampooing, and in the case of straight hair, wavy or curly hair is not substantially generated as a result of shampooing.

In the present invention, "hair deformation" means deformation of hair, which is not caused by the cleavage and recombination of the S-S bond of proteins in hair, and it includes deformation of straight hair into curly hair or the like, and also, deformation of hair which has been deformed into wavy or curly hair, or naturally curly hair, to straight hair.

### [Component (A): glyoxylic acid, or a hydrate or salt thereof]

The component (A) includes not only glyoxylic acid, but also a hydrate of the glyoxylic acid and a salt of the glyoxylic acid. An example of the hydrate of the glyoxylic acid is a glyoxylic acid monohydrate. Examples of the salt of the glyoxylic acid include an alkaline metal salt of the glyoxylic acid and an alkaline-earth metal salt of the glyoxylic acid. Examples of the alkaline metal salt include a lithium salt, a sodium salt, and a potassium salt. Examples of the alkaline-earth metal salt include a magnesium salt and a calcium salt.

From the viewpoint of achieving a more significant change in the shape of hair after the treatment of hair with the hair treatment agent of the present invention, achieving more excellent hair washing resistance of the shape of hair, achieving a more significant change in the shape of hair upon semi-permanent re-deformation of the shape of hair by heating, and also achieving more excellent hair washing resistance of the shape of hair after completion of the re-deformation, the content of the component (A) in the agent for hair deforming treatment of the present invention is, in terms of glyoxylic acid, preferably 1 mass% or more, more preferably 2 mass% or more, even more preferably 2.5 mass% or more, and further preferably 3 mass% or more, and in addition to the aforementioned viewpoint, also from the viewpoint of suppression of irritation to the skin, the content of the component (A) is, in terms of glyoxylic acid, preferably 30 mass% or less, more preferably 25 mass% or less, even more preferably 20 mass% or less, even more preferably 15 mass% or less, and even more preferably 12 mass% or less.

### [Component (B): a compound represented by formula (1)]

The component (B) is a compound represented by the following formula (1) : 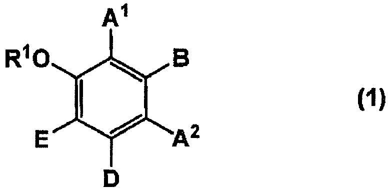 wherein
R¹ represents a hydrogen atom or a methyl group,
A¹ and A², which may be the same or different, each represent a hydrogen atom, a straight-chain or branched-chain alkyl group or alkenyl group containing from 1 to 12 carbon atoms, an optionally substituted aralkyl group or arylalkenyl group containing from 7 to 12 carbon atoms, a straight-chain or branched-chain alkoxy group or alkenyloxy group containing from 1 to 6 carbon atoms, a halogen atom, or -CO-R² (wherein R² represents a straight-chain or branched-chain alkyl group or alkenyl group containing from 1 to 12 carbon atoms, an optionally substituted aralkyl group or arylalkenyl group containing from 7 to 12 carbon atoms, or an optionally substituted aromatic hydrocarbon group containing from 6 to 12 carbon atoms),
B represents a hydrogen atom, a straight-chain or branched-chain alkyl group or alkenyl group containing from 1 to 12 carbon atoms, an optionally substituted aralkyl group or arylalkenyl group containing from 7 to 12 carbon atoms, -OR³, or -COOR³ (wherein R³ represents a hydrogen atom, or a straight-chain or branched-chain alkyl group or alkenyl group containing from 1 to 6 carbon atoms),
D represents a hydrogen atom, a hydroxy group, a methyl group, or a straight-chain or branched-chain alkoxy group or alkenyloxy group containing from 1 to 12 carbon atoms, and
E represents a hydrogen atom, a hydroxy group, a straight-chain or branched-chain alkyl group or alkenyl group containing from 1 to 6 carbon atoms, or a straight-chain or branched-chain alkoxy group or alkenyloxy group containing from 1 to 6 carbon atoms,
provided that two or three of A¹, A², B and E are hydrogen atoms, and the remaining groups do not include sulfo groups, and that when D is a hydrogen atom or a methyl group, A¹ and B, or A² and B, together with two carbon atoms adjacent to them, form a benzene ring optionally substituted with a hydroxy group.

In the formula (1), when the aralkyl group, arylalkenyl group, or aromatic hydrocarbon group has a substituent, examples of the substituent include a hydroxy group, a straight-chain or branched-chain alkyl group or alkenyl group containing from 1 to 6 carbon atoms, and an alkoxy group containing from 1 to 12 carbon atoms. In addition, the number of carbon atoms contained in the aralkyl group, arylalkenyl group, or aromatic hydrocarbon group indicates the total number of carbon atoms including the number of carbon atoms contained in substituents.

Examples of the straight-chain or branched-chain alkyl group or alkenyl group containing from 1 to 6 carbon atoms, which is represented by R³, include a methyl group, an ethyl group, an n-propyl group, an isopropyl group, an n-butyl group, an isobutyl group, a sec-butyl group, a tert-butyl group, an n-pentyl group, an isopentyl group, a neopentyl group, a 1-methylpentyl group, an n-hexyl group, an isohexyl group, a vinyl group, an allyl group, a butenyl group, a hexenyl group.

An example of the straight-chain or branched-chain alkoxy group or alkenyloxy group containing from 1 to 6 carbon atoms, which is represented by A¹, A² or E, is a group formed by binding an oxygen atom to the above described alkyl group or alkenyl group containing from 1 to 6 carbon atoms.

Examples of the straight-chain or branched-chain alkyl group or alkenyl group containing from 1 to 12 carbon atoms, which is represented by A¹, A², R² or B, include the above described alkyl group or alkenyl group containing from 1 to 6 carbon atoms, an n-heptyl group, a 2,4-dimethylpentyl group, a 1-n-propylbutyl group, an n-octyl group, a 2-ethylhexyl group, an n-nonyl group, a 1-methylnonyl group, an n-decyl group, a 3,7-dimethyloctyl group, a 2-isopropyl-5-methylhexyl group, an n-undecyl group, an n-dodecyl group, and a decenyl group.

An example of the straight-chain or branched-chain alkoxy group or alkenyloxy group containing from 1 to 12 carbon atoms, which is represented by D, is a group formed by binding an oxygen atom to the above described alkyl group or alkenyl group containing from 1 to 12 carbon atoms.

Examples of the optionally substituted aralkyl group or arylalkenyl group containing from 7 to 12 carbon atoms, which is represented by A¹, A², R² or B, include a benzyl group, a hydroxybenzyl group, a dihydroxybenzyl group, a phenylethyl group, a phenylethenyl group, a hydroxyphenylethyl group, a dihydroxyphenylethyl group, a hydroxyphenylethenyl group, a dihydroxyphenylethenyl group, a phenylpropyl group, a phenylpropenyl group, a phenylbutyl group, a phenylbutenyl group, a phenylpentyl group, a phenylpentenyl group, a phenylhexyl group, and a phenylhexenyl group.

Examples of the optionally substituted aromatic hydrocarbon group containing from 6 to 12 carbon atoms, which is represented by R², include a phenyl group, a hydroxyphenyl group, a dihydroxyphenyl group, a trihydroxyphenyl group, a naphthyl group, a hydroxynaphthyl group, and a dihydroxynaphthyl group.

Examples of the halogen atom represented by A¹ or A² include a fluorine atom, a chlorine atom, and a bromine atom.

Specific examples of the compound represented by the formula (1) include a resorcin derivative represented by the following formula (1-1), a benzophenone derivative represented by the following formula (1-2), and a naphthol derivative represented by the following formula (1-3-a) or (1-3-b): 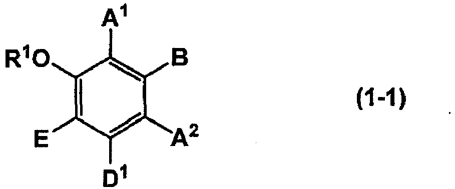 wherein R¹, A¹, A², B and E are defined as those described above, and D¹ represents a hydroxy group or a methoxy group, 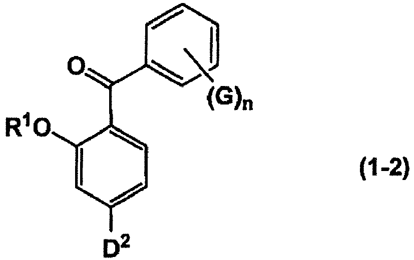 wherein R¹ is as defined above, D² represents a hydroxy group, or an alkoxy group containing from 1 to 12 carbon atoms, G represents a hydroxy group, a straight-chain or branched-chain alkyl group or alkenyl group containing from 1 to 6 carbon atoms, or an alkoxy group containing from 1 to 6 carbon atoms, and n represents an integer of from 0 to 2, and 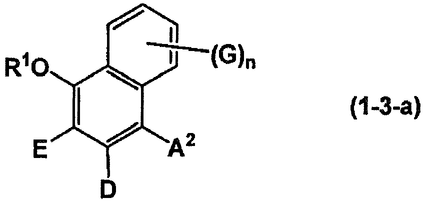 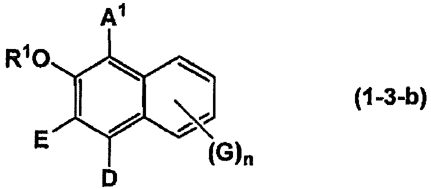 wherein R¹, A¹, E, D, G and n are as defined above.

Preferred examples of the compound represented by the formula (1-1) include the following compounds (1-1-1) to (1-1-3).

### (1-1-1) An m-dimethoxybenzene derivative represented by the following formula (1-1-1):

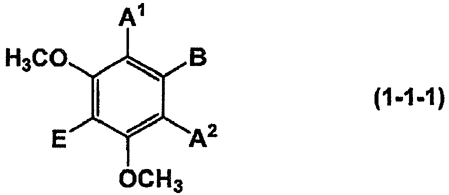

wherein A¹, A², B and E are as defined above.

A¹ and A² each represent, preferably a hydrogen atom, or a straight-chain or branched-chain alkyl group or alkenyl group containing from 1 to 4 carbon atoms, and more preferably a hydrogen atom.

B represents, preferably a hydrogen atom, an alkyl group or alkenyl group containing from 1 to 4 carbon atoms, an optionally substituted arylalkenyl group containing from 7 to 10 carbon atoms, or a hydroxy group, and more preferably a hydrogen atom, an optionally substituted arylalkenyl group containing from 7 to 10 carbon atoms, or a hydroxy group.

E represents, preferably a hydrogen atom, or a straight-chain or branched-chain alkyl group or alkenyl group containing from 1 to 4 carbon atoms, and more preferably a hydrogen atom.

Examples of the compound corresponding to (1-1-1) include 1,3-dimethoxybenzene, 3,5-dimethoxyphenol, 2,6-dimethoxyphenol, and 5-(hydroxyphenylethenyl)-1,3-dimethoxybenzene (trivial name: pterostilbene).

### (1-1-2) An m-methoxyphenol derivative represented by the following formula (1-1-2):

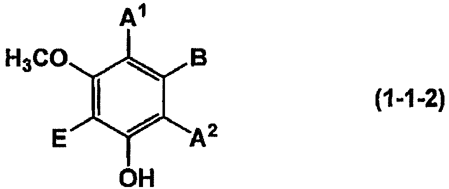

wherein A¹, A², B and E are as defined above.

A¹ and A² each represent, preferably a hydrogen atom, a straight-chain or branched-chain alkyl group or alkenyl group containing from 1 to 12 carbon atoms, or an optionally substituted aralkyl group or arylalkenyl group containing from 7 to 12 carbon atoms, and more preferably a hydrogen atom, a straight-chain or branched-chain alkyl group containing from 1 to 6 carbon atoms, or an optionally substituted arylalkenyl group containing from 7 to 10 carbon atoms.

B represents, preferably a hydrogen atom, a straight-chain or branched-chain alkyl group or alkenyl group containing from 1 to 12 carbon atoms, an optionally substituted aralkyl group or arylalkenyl group containing from 7 to 12 carbon atoms, or -OR³ (wherein R³ represents a hydrogen atom, or a straight-chain or branched-chain alkyl group or alkenyl group containing from 1 to 6 carbon atoms), more preferably a hydrogen atom, an alkyl group or alkenyl group containing from 1 to 4 carbon atoms, an optionally substituted arylalkenyl group containing from 7 to 10 carbon atoms, or a hydroxy group, and even more preferably a hydrogen atom, an optionally substituted arylalkenyl group containing from 7 to 10 carbon atoms, or a hydroxy group.

E represents, preferably a hydrogen atom, a hydroxy group, a straight-chain or branched-chain alkyl group or alkenyl group containing from 1 to 4 carbon atoms, or a straight-chain or branched-chain alkoxy group or alkenyloxy group containing from 1 to 4 carbon atoms, and more preferably a hydrogen atom or a hydroxy group.

Examples of the compound corresponding to (1-1-2) include 3-methoxyphenol, 5-methoxy resorcin, 3-methoxybenzene-1,2-diol, 4-butyl-3-methoxyphenol, 3-methoxy-4-(1-phenylethyl)phenol, and 5-(4-hydroxyphenylethenyl)-1-hydroxy-3-methoxybenzene (trivial name: Pinostilbene) .

### (1-1-3) A resorcin derivative represented by the following formula (1-1-3) :

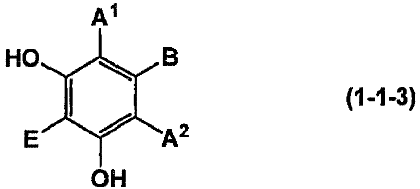

wherein A¹, A², B and E are as defined above.

Examples of the resorcin derivative include those represented by the following formulae (i) or (ii). 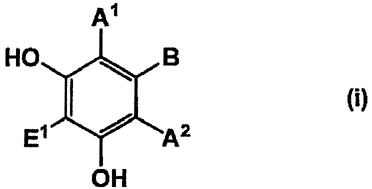 wherein A¹, A² and B are as defined above, E¹ represents a hydroxy group, a straight-chain or branched-chain alkyl group or alkenyl group containing from 1 to 6 carbon atoms, or a straight-chain or branched-chain alkoxy group or alkenyloxy group containing from 1 to 6 carbon atoms.

A¹ and A² each represent, preferably a hydrogen atom, or a straight-chain or branched-chain alkyl group or alkenyl group containing from 1 to 12 carbon atoms, and more preferably a hydrogen atom.

B represents, preferably a hydrogen atom, an optionally substituted aralkyl group or arylalkenyl group containing from 7 to 12 carbon atoms, -OR³ (wherein R³ represents a hydrogen atom, or a straight-chain or branched-chain alkyl group or alkenyl group containing from 1 to 4 carbon atoms).

E¹ preferably represents a hydroxy group, a straight-chain or branched-chain alkyl group or alkenyl group containing from 1 to 4 carbon atoms, or a straight-chain or branched-chain alkoxy group or alkenyloxy group containing from 1 to 4 carbon atoms.

Examples of the resorcin derivative represented by the formula (i) include:
2-alkyl resorcin, such as 2-methyl resorcin, 2-ethyl resorcin, or 2-propyl resorcin;
pyrogallol;
2-alkoxy resorcin, such as 2-methoxy resorcin;
gallic acid, and gallic acid ester, such as methyl gallate, ethyl gallate, propyl gallate, or butyl gallate; and
5-(phenylethenyl) 2-isopropyl resorcin. 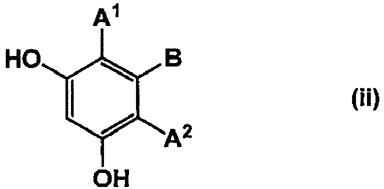
wherein A¹, A², B and E are as defined above.

The resorcin derivative represented by the formula (ii) is more preferably a resorcin derivative represented by the following formula (ii-1) or (ii-2). 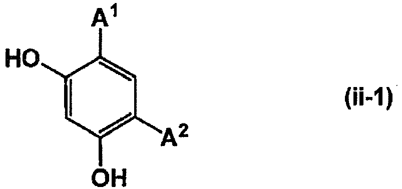 wherein A¹ and A² are as defined above.

Examples of the resorcin derivative represented by the formula (ii-1) include:
4-alkyl resorcin, such as 4-methyl resorcin, 4-ethyl resorcin, 4-propyl resorcin, 4-isopropyl resorcin, 4-butyl resorcin (trivial name: Rucinol), 4-isobutyl resorcin, 4-sec-butyl resorcin, 4-tert-butyl resorcin, 4-pentyl resorcin, 4-isopentyl resorcin, 4-sec-pentyl resorcin, 4-tert-pentyl resorcin, 4-neopentyl resorcin, 4-hexyl resorcin, 4-isohexyl resorcin, 4-heptyl resorcin, 4-octyl resorcin, 4-(2-ethylhexyl) resorcin, 4-nonyl resorcin, 4-decyl resorcin, 4-undecyl resorcin, or 4-dodecyl resorcin;
4-alkenyl resorcin, such as 4-vinyl resorcin, 4-allyl resorcin, 4-butenyl resorcin, 4-hexenyl resorcin, or 4-decenyl resorcin;
4-aralkyl resorcin, such as 4-benzyl resorcin, 4-(1-phenylethyl) resorcin (trivial name: Symwhite 377), 4-(2-phenylethyl) resorcin, or 4-(3-phenylpropyl) resorcin;
4-hydroxyaralkyl resorcin, such as 4-(4-hydroxybenzyl) resorcin, 4-(2,4-dihydroxybenzyl) resorcin, 4-(4-hydroxyphenylethyl) resorcin, or 4-(2,4-dihydroxyphenylethyl) resorcin;
4-arylalkenyl resorcin, such as 4-(1-phenylethenyl) resorcin or 4-(3-phenylpropenyl) resorcin;
4-hydroxyarylalkenyl resorcin, such as 4-(4-hydroxyphenylethenyl) resorcin or 4-(2,4-dihydroxyphenylethenyl) resorcin;
4-(1-methylnaphthyl) resorcin;
4-alkoxy resorcin, such as 4-methoxy resorcin, 4-ethoxy resorcin, 4-isopropoxy resorcin, 4-propoxy resorcin, 4-butoxy resorcin, 4-sec-butoxy resorcin, 4-tert-butoxy resorcin, or 4-pentoxy resorcin;
halogenated resorcin, such as 4-chloro resorcin or 4-bromo resorcin;
4-alkanoyl resorcin, such as 4-acetyl resorcin, 4-propanoyl resorcin, 4-butanoyl resorcin, 4-pentanoyl resorcin, or 4-hexanoyl resorcin; and
4-arylalkanoyl resorcin, such as 4-phenylethanoyl resorcin, 4-phenylpropanoyl resorcin, 4-phenylbutanoyl resorcin, 4-phenylpentanoyl resorcin, 4-phenylhexanoyl resorcin, or 3-(hydroxyphenyl)-1-(2,4-dihydroxyphenyl)propen-1-one (trivial name: Isoliquiritigenin). 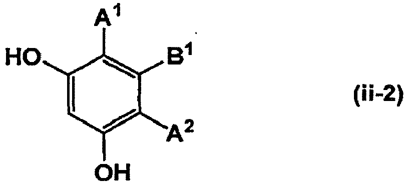
wherein A¹ and A² are as defined above, and B¹ represents a straight-chain or branched-chain alkyl group or alkenyl group containing from 1 to 12 carbon atoms, an optionally substituted aralkyl group or arylalkenyl group containing from 7 to 12 carbon atoms, -OR³, or -COOR³ (wherein R³ represents a hydrogen atom, or a straight-chain or branched-chain alkyl group or alkenyl group containing from 1 to 6 carbon atoms).

The resorcin derivative represented by the formula (ii-2) is more preferably a resorcin derivative represented by the following formula (ii-2-a) or (ii-2-b). 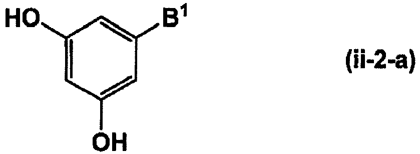 wherein B¹ is as defined above.

Examples of the resorcin derivative represented by the formula (ii-2-a) include:
5-alkyl resorcin, such as 5-methyl resorcin, 5-ethyl resorcin, 5-propyl resorcin, 5-isopropyl resorcin, 5-butyl resorcin, 5-isobutyl resorcin, 5-sec-butyl resorcin, 5-tert-butyl resorcin, 5-pentyl-resorcin (trivial name: Olivetol), 5-isopentyl resorcin, 5-neopentyl resorcin, 5-hexyl resorcin, 5-isohexyl resorcin, 5-heptyl resorcin, 5-octyl resorcin, 5-(2-ethylhexyl) resorcin, 5-nonyl resorcin, 5-decyl resorcin, 5-undecyl resorcin, or 5-dodecyl resorcin;
5-alkenyl resorcin, such as 5-vinyl resorcin, 5-allyl resorcin, 5-butenyl resorcin, 5-hexenyl resorcin, or 5-decenyl resorcin;
phloroglucinol;
5-alkoxybenzene-1,3-diol, such as 5-ethoxybenzene-1,3-diol, 5-propoxybenzene-1,3-diol, or 5-butoxybenzene-1,3-diol;
3,5-dihydroxybenzoic acid;
3,5-dihydroxybenzoic acid ester, such as methyl 3,5-dihydroxybenzoate, ethyl 3,5-dihydroxybenzoate, propyl 3,5-dihydroxybenzoate, butyl 3,5-dihydroxybenzoate, pentyl 3,5-dihydroxybenzoate, or hexyl 3,5-dihydroxybenzoate;
5-aralkyl resorcin, such as 5-benzyl resorcin, 5-(1-phenylethyl) resorcin, 5-(2-phenylethyl) resorcin, or 5-(phenylpropyl) resorcin;
5-hydroxyaralkyl resorcin, such as 5-(4-hydroxybenzyl) resorcin, 5-(2,4-dihydroxybenzyl) resorcin, 5-(hydroxyphenylethyl) resorcin (trivial name: Dihydro-resveratrol), or 5-(2,4-dihydroxyphenylethyl) resorcin;
5-arylalkenyl resorcin, such as 5-(phenylethenyl) resorcin (trivial name: Pinosylvin) or 5-(phenylpropenyl) resorcin; and
5-hydroxyarylalkenyl resorcin, such as 5-(4-hydroxyphenylethenyl) resorcin (trivial name: Resveratrol), 5-(4-methoxyphenylethenyl) resorcin (trivial name: 4-MethoxyResveratrol), 5-(2,4-dihydroxyphenylethenyl) resorcin (trivial name: Oxyresveratrol), 5-(2-methoxy-4-hydroxyphenylethenyl) resorcin (trivial name: Gnetucleistol D), 5-(3,4-dimethoxyphenylethenyl) resorcin (trivial name: Gnetucleistol E), 5-(3-hydroxy-4-methoxyphenylethenyl) resorcin (trivial name: Rhapontigenin), 5-(4-hydroxy-3-methoxyphenylethenyl) resorcin (trivial name: Isorhapontigenin), or 5-(dihydroxyphenylethenyl) resorcin (trivial name: Piceatannol). 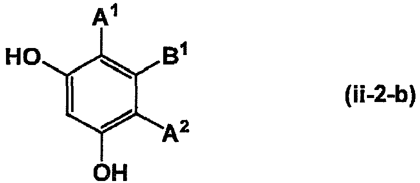
wherein A¹, A² and B¹ are as defined above.

A¹ and A² each preferably represent a hydrogen atom, a straight-chain or branched-chain alkyl group or alkenyl group containing from 1 to 4 carbon atoms, or an alkoxy group or alkenyloxy group containing from 1 to 4 carbon atoms.

Examples of the resorcin derivative represented by the formula (ii-2-b) include:
2-alkylbenzene-1,3,5-triol, such as 2-methylbenzene-1,3,5-triol, 2-ethylbenzene-1,3,5-triol, 2-propylbenzene-1,3,5-triol, 2-butylbenzene-1,3,5-triol, 2-hexylbenzene-1,3,5-triol, 2-octylbenzene-1,3,5-triol, or 2-dodecylbenzene-1,3,5-triol;
2-aralkyl-l,3,5-triol, such as 2-benzylbenzene-1,3,5-triol, 2-(phenylethyl)benzene-1,3,5-triol, or 2-(phenylpropyl)benzene-1,3,5-triol;
phloroglucin acid ester, such as 2-acetylbenzene-1,3,5-triol, 2-propanoylbenzene-1,3,5-triol, 2-butanoylbenzene-1,3,5-triol, 2-phenylethanoylbenzene-1,3,5-triol, 2-hydroxyphenyl-1-(benzene-2,4,6-triol)ethan-1-one, 3-hydroxyphenyl-1-(benzene-2,4,6-triol)propan-1-one (trivial name: Phloretin), 4-hydroxyphenyl-1-(benzene-2,4,6-triol)butan-1-one, 2-benzoylbenzene-1,3,5-triol, 2-(hydroxybenzoyl)benzene-1,3,5-triol, 2-(3,5-dihydroxybenzoyl)benzene-1,3,5-triol, or 2-(2,4-dihydroxybenzoyl)benzene-1,3,5-triol; and
3,5-dihydroxybenzoic acid ester, such as 3,5-dihydroxy-2-methylbenzoic acid, methyl 3,5-dihydroxy-2-methylbenzoate, 3,5-dihydroxy-2-ethylbenzoic acid, methyl 3,5-dihydroxy-2-ethylbenzoate, 3,5-dihydroxy-2-propylbenzoic acid, methyl 3,5-dihydroxy-2-propylbenzoate, 3,5-dihydroxy-2-butylbenzoic acid, or methyl 3,5-dihydroxy-2-butylbenzoate.

Examples of the benzophenone derivative represented by the formula (1-2) include 4-benzoyl resorcin (trivial name: Benzophenone-1), 4-(hydroxybenzoyl) resorcin, 4-(dihydroxybenzoyl) resorcin, 4-(2,4-dihydroxybenzoyl) resorcin (trivial name: Benzophenone-2), 4-(methylbenzoyl) resorcin, 4-(ethylbenzoyl) resorcin, 4-(dimethylbenzoyl) resorcin, 4-(diethylbenzoyl) resorcin, 4-naphthoyl resorcin, 2-hydroxy-4-methoxybenzophenone (trivial name: Benzophenone-3), 2,2'-dihydroxy-4,4'-dimethoxybenzophenone (trivial name: Benzophenone-6), 2,2'-dihydroxy-4-methoxybenzophenone (trivial name: Benzophenone-8), 2-hydroxy-4-methoxy-4'-methylbenzophenone (trivial name: Benzophenone-10), and 2-hydroxy-4-octyloxybenzophenone (trivial name: Benzophenone-12).

The naphthol derivative represented by the formula (1-3-a) or (1-3-b) is preferably a naphthol derivative, wherein, in the formula (1-3-a) or (1-3-b), R¹ represents a hydrogen atom, or an alkyl group or alkenyl containing from 1 to 4 carbon atoms, and more preferably, a naphthol derivative, wherein, in the formula (1-3-a) or (1-3-b), R¹ represents a hydrogen atom.

Moreover, a naphthol derivative, wherein A¹ and A² each represent a hydrogen atom, a hydroxy group, a straight-chain or branched-chain alkyl group containing from 1 to 4 carbon atoms, or an alkoxy group containing from 1 to 4 carbon atoms, is preferable; and a naphthol derivative, wherein A¹ and A² each represent a hydrogen atom or a hydroxy group, is more preferable.

Furthermore, a naphthol derivative, wherein D represents a hydrogen atom, a hydroxy group, a straight-chain or branched-chain alkyl group containing from 1 to 4 carbon atoms, or an alkoxy group containing from 1 to 4 carbon atoms, is preferable.

Further, a naphthol derivative, wherein E represents a hydrogen atom, a hydroxy group, or an alkyl group containing from 1 to 4 carbon atoms or an alkoxy group containing from 1 to 4 carbon atoms, is preferable.

Examples of such a compound include 1-naphthol, 2-naphthol, 3-methylnaphthalen-1-ol, naphthalene-1,4-diol, naphthalene-1,5-diol, and naphthalene-1,8-diol.

Among the compounds represented by the formula (1), the m-dimethoxybenzene derivative represented by the formula (1-1-1), the resorcin derivative represented by the formula (1-1-3), the benzophenone derivative represented by the formula (1-2), and the naphthol derivative represented by the formula (1-3-a) or (1-3-b) are preferable. Moreover, 2-methyl resorcin, 4-chloro resorcin, 4-alkyl resorcin, 4-aralkyl resorcin, 4-acylated resorcin, 5-alkyl resorcin, 5-aralkyl resorcin, 5-hydroxyarylalkenyl resorcin, phloroglucin acid ester, gallic acid, and gallic acid ester are preferable. Furthermore, 4-butyl resorcin (trivial name: Rucinol), 4-(1-phenylethyl) resorcin (trivial name: Symwhite 377), 5-(hydroxyphenylethenyl) resorcin (trivial name: resveratrol), 3-hydroxyphenyl-1-(benzene-2,4,6-triol)propan-1-one (trivial name: Phloretin), 4-(2,4-dihydroxybenzoyl) resorcin (trivial name: Benzophenone-2), 5-(hydroxyphenylethenyl)-1,3-dimethoxybenzene (trivial name: Pterostilbene), and 1-naphthol are preferable. Further, 2-methyl resorcin, 4-chloro resorcin, 1-naphthol, 4-n-butyl resorcinol, 4-phenyl resorcinol, 5-(hydroxyphenylethenyl) resorcin, 3-hydroxyphenyl-1-(benzene-2,4,6-triol)propan-1-one, and 4-(2,4-dihydroxybenzoyl) resorcin are preferable.

The molecular weight of the compound represented by the formula (1) is preferably 120 or more, and from the viewpoint of permeability into hair, it is preferably 1,000 or less, more preferably 500 or less, and even more preferably 300 or less.

The component (B) can be used alone or in combination of two or more components. The use of two or more components is more preferable. From the viewpoint of achieving a more significant change in the shape of hair after the treatment of hair with the hair treatment agent of the present invention, achieving more excellent hair washing resistance of the shape of hair, achieving a more significant change in the shape of hair upon semi-permanent re-deformation of the shape of hair by heating, and also achieving more excellent hair washing resistance of the shape of hair after completion of the re-deformation, the content of the component (B) in the agent for hair deforming treatment of the present invention is preferably 0.2 mass% or more, more preferably 0.5 mass% or more, even more preferably 1 mass% or more, further preferably 5 mass% or more, and still further preferably 10 mass% or more, and it is preferably 30 mass% or less, more preferably 25 mass% or less, even more preferably 23 mass% or less, and further preferably 20 mass% or less.

From the viewpoint of, because of a condensate of the component (A) and the component (B) formed in hair, achieving a more significant change in the shape of hair after the treatment of hair with the hair treatment agent of the present invention, achieving more excellent hair washing resistance of the shape of hair, achieving a more significant change in the shape of hair upon semi-permanent re-deformation of the shape of hair by heating, and also achieving more excellent hair washing resistance of the shape of hair after completion of the re-deformation, the molar ratio of the content of the component (B) to the content of the component (A) in the agent for hair deforming treatment of the present invention, (B)/(A), is preferably 0.1 or more, more preferably 0.3 or more, even more preferably 0.5 or more, and further preferably 0.7 or more, and it is preferably 5 or less, more preferably 2.5 or less, even more preferably 2 or less, further preferably 1.5 or less, and still further preferably 1.2 or less.

### [Component (C): water]

The agent for hair deforming treatment of the present invention uses water as a medium. Moreover, in addition to water, lower alcohol containing from 1 to 3 carbon atoms, such as methanol or ethanol, can be used in combination with water, as necessary. In this case, the content of the lower alcohol containing from 1 to 3 carbon atoms in the agent for hair deforming treatment of the present invention is preferably 60 mas% or less, more preferably 40 mass% or less, even more preferably 30 mass% or less, further preferably 20 mass% or less, still further preferably 15 mass% or less, and still further preferably 10 mass% or less. In addition, it is preferably 0.1 mass% or more.

The agent for hair deforming treatment of the present invention may be either one-part agent, or a multi-part agent such as a two-part agent. From the viewpoint of ameliorating the permeability of the component (A) and the component (B) into hair and increasing the effects of the present invention, a multi-part agent comprising the component (A) and the component (B) in respective parts, and further, a two-part agent is more preferable. In the case of such a multi-part agent, a part comprising the component (B) is defined as a first agent and a part comprising the component (A) is defined as a second agent in the present invention.

In the case of the multi-part agent, the content of the component (B) in the first agent is preferably 0.2 mass% or more, more preferably 0.5 mass% or more, even more preferably 1 mass% or more, further preferably 5 mass% or more and still further preferably 10 mass% or more, and it is preferably 30 mass% or less, more preferably 25 mass% or less, even more preferably 23 mass% or less, and further preferably 20 mass% or less.

In the case of the multi-part agent, the content of the component (A) in the second agent is, in terms of glyoxylic acid, preferably 1 mass% or more, more preferably 2 mass% or more, even more preferably 2.5 mass% or more, and further preferably 3 mass% or more, and it is preferably 30 mass% or less, more preferably 25 mass% or less, even more preferably 20 mass% or less, further preferably 15 mass% or less, and still further preferably 12 mass% or less.

When the first agent, the second agent and the like are mixed in the multi-part agent and the obtained mixture is then used, the mixing ratio is not particularly limited. It is preferable that the composition obtained after the mixing of the parts become the agent for hair deforming treatment of the present invention. Accordingly, in the case of the multi-part agent, the contents of individual components are the contents thereof in the composition obtained after the mixing of the parts, unless otherwise specified. In addition, upon production of, the multi-part agent can comprise the component (C) as a solvent for each part, and the component (C) is preferably present both in the first agent and in the second agent.

In the case of the one-part agent, from the viewpoint of the permeability (A) into hair, the pH of the agent for hair deforming treatment of the present invention is preferably 4 or less, more preferably 3 or less, even more preferably 2.5 or less, and further preferably 2 or less. In addition, in the case of the one-part agent, from the viewpoint of suppression of hair damage and suppression of irritation to the skin, the pH of the present agent for hair deforming treatment is preferably 1 or more, more preferably 1.2 or more, and even more preferably 1.5 or more. In the case of the multi-part agent, the pH of the part comprising the component (A), namely, the pH of the second agent is preferably set in the above described range, and more preferably, the pH of the mixture obtained after mixing all of the parts is set in the above described range. In the present application, the pH of the hair treatment agent indicates a value obtained by directly measuring the pH of the agent for hair deforming treatment at a room temperature (25°C) using a pH meter, without dilution and the like of the agent for hair deforming treatment.

In order to adjust the pH of the agent for hair deforming treatment to be in the above described range, a pH adjuster can be used, as appropriate. Examples of the pH adjuster as an alkali agent that can be used include: ammonia or a salt thereof; alkanolamine such as monoethanolamine, isopropanolamine, 2-amino-2-methylpropanol or 2-aminobutanol, or a salt thereof; alkanediamine such as 1,3-propanediamine, or a salt thereof; carbonates such as guanidine carbonate, sodium carbonate, potassium carbonate, sodium hydrogen carbonate, or potassium hydrogen carbonate; and hydroxides such as sodium hydroxide or potassium hydroxide. Moreover, as an acid agent, inorganic acids such as hydrochloric acid or phosphoric acid, hydrochlorides such as monoethanolamine hydrochloride, phosphates such as monopotassium dihydrogen phosphate or disodium monohydrogen phosphate, or organic acids other than the component (A), such as lactic acid or malic acid, can be used.

From the viewpoint of improving the touch feeling of hair after completion of a hair treatment and further improving the effects of the present invention, the agent for hair deforming treatment of the present invention preferably comprises a cationic surfactant. The cationic surfactant is preferably a mono-long-chain-alkyl quaternary ammonium salt having an alkyl group containing from 8 to 24 carbon atoms and three alkyl groups containing from 1 to 4 carbon atoms.

Preferably, at least one mono-long-chain-alkyl quaternary ammonium surfactant is selected from the group consisting of compounds represented by the following formula: 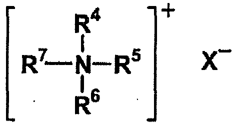 wherein R⁴ represents a saturated or unsaturated, straight-chain or branched-chain alkyl group containing from 8 to 22 carbon atoms, R⁸-CO-NH-(CH₂)ₘ-, or R⁸-CO-O-(CH₂)ₘ-(wherein R⁸ represents a saturated or unsaturated, straight-chain or branched-chain alkyl chain containing from 7 to 21 carbon atoms, and m represents an integer from 1 to 4); R⁵, R⁶ and R⁷ each independently represent an alkyl group containing from 1 to 4 carbon atoms or a hydroxylalkyl group containing from 1 to 4 carbon atoms, and X⁻ represents a chloride ion, a bromide ion, a methosulfate ion, or an ethosulfate ion.

Examples of a preferred cationic surfactant include long-chain quaternary ammonium compounds such as cetyltrimethylammonium chloride, myristyltrimethylammonium chloride, behentrimonium chloride, cetyltrimethylammonium bromide, and stearamidopropyl trimonium chloride. These compounds can be used alone, or can also be used in the form of a mixture thereof.

The content of the cationic surfactant in the agent for hair deforming treatment of the present invention is preferably 0.05 mass% or more, and more preferably 0.1 mass% or more, and it is preferably 10 mass% or less, and more preferably 5 mass% or less. When the agent for hair deforming treatment is a multi-part agent, the multi-part agent may comprise the cationic surfactant in the first agent, or in the second agent, or both of the first and second agents.

Furthermore, from the viewpoint of improving the touch feeling of hair after completion of a hair treatment and achieving good manageability, the agent for hair deforming treatment of the present invention preferably comprises silicone. As such silicone, dimethylpolysiloxane and amino-modified silicone are preferable.

As dimethylpolysiloxane, either a cyclic or acyclic dimethylpolysiloxane polymer can be used. Examples include SH200 series, BY22-019, BY22-020, BY11-026, B22-029, BY22-034, BY22-050A, BY22-055, BY22-060, BY22-083 and FZ-4188 (all of which are manufactured by Dow Corning Toray Co., Ltd.), and KF-9088, KM-900 series, MK-15H and MK-88 (all of which are manufactured by Shin-Etsu Chemical Co., Ltd.).

As amino-modified silicone, all silicones having an amino group or an ammonium group can be used. Examples include amino-modified silicone oil, all or a part of terminal hydroxy groups of which are sealed with methyl groups and the like, and amodimethicone, the terminus of which is not sealed. As preferred amino-modified silicone, a compound represented by the following formula can be used, for example: 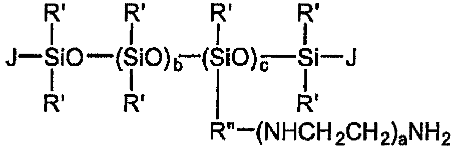 wherein R' represents a hydrogen atom, a hydroxy group, or R^{x}, wherein R^{x} represents a substituted or unsubstituted monovalent hydrocarbon group containing from 1 to 20 carbon atoms; J represents R^{x}, R"-(NHCH₂CH₂)ₐNH₂, OR^{x}, or a hydroxy group, wherein R" represents a divalent hydrocarbon group containing from 1 to 8 carbon atoms; a represents a number of from 0 to 3; and b and c each represent a number, in which the sum thereof is, at a number average, 10 or more and less than 20,000, preferably 20 or more and less than 3,000, more preferably 30 or more and less than 1,000, and even more preferably 40 or more and less than 800.

Specific examples of a commercially available product of preferred amino-modified silicone include: amino-modified silicone oils such as SF8452C and SS3551 (both of which are manufactured by Dow Corning Toray Co., Ltd.), and KF-8004, KF-867S and KF-8015 (all of which are manufactured by Shin-Etsu Chemical Co., Ltd.); and amodimethicone emulsions such as SM8704C, SM8904, BY22-079, FZ-4671 and FZ4672 (all of which are manufactured by Dow Corning Toray Co., Ltd.) .

The content of the silicone in the agent for hair deforming treatment of the present invention is preferably 0.1 mass% or more, more preferably 0.2 mass% or more, and even more preferably 0.5 mass% or more, and it is preferably 20 mass% or less, more preferably 10 mass% or less, and even more preferably 5 mass% or less. When the agent for hair deforming treatment is a multi-part agent, the multi-part agent may comprise the silicone in the first agent or in the second agent, or both of the first and second agents.

Further, from the viewpoint of improving the touch feeling of hair after completion of a hair treatment, the agent for hair deforming treatment of the present invention preferably comprises a cationic polymer.

The cationic polymer means a polymer having a cationic group or a group that can be ionized to such a cationic group. An amphoteric polymer, which can be cationic as a whole, is also included in the cationic polymer. That is to say, examples of the cationic polymer include water-soluble cationic polymers, which comprise an amino group or an ammonium group in the side chains thereof, or which comprise, as constitutional unit, a diallyl quaternary ammonium salt, such as a cationized cellulose derivative, cationic starch, a cationized guar gum derivative, a polymer or copolymer of diallyl quaternary ammonium salts, and a quaternary polyvinyl pyrrolidone derivative. In terms of effects such as touch feeling softness, smoothness, and easy to run fingers through hair obtained during rinsing or shampooing, manageability and moisture retention during drying, and the stability of the agent, among the aforementioned cationic polymers, a polymer comprising, as a constitutional unit, a diallyl quaternary ammonium salt, a quaternary polyvinyl pyrrolidone derivative, and a cationized cellulose derivative are preferable; and a polymer or copolymer of diallyl quaternary ammonium salts, and a cationized cellulose derivative are more preferable.

Specific examples of a preferred polymer or copolymer of diallyl quaternary ammonium salts include a dimethyldiallyl ammonium chloride polymer (Polyquaternium-6, for example, Marquardt 100; Lubrizol Advanced Materials), a dimethyldiallyl ammonium chloride/acrylic acid copolymer (Polyquaternium-22, for example, Marquardt 280 and Marquardt 295; Lubrizol Advanced Materials), and a dimethyldiallyl ammonium chloride/acrylamide copolymer (Polyquaternium-7, for example, Marquardt 550; Lubrizol Advanced Materials).

Specific examples of a preferred quaternary polyvinyl pyrrolidone derivative include polymers obtained by polymerizing a vinyl pyrrolidone copolymer with dimethylaminoethyl methacrylate (Polyquaternium-11, for example, GAFQUAT 734, GAFQUAT 755, and GAFQUAT 755N (all of which are manufactured by Ashland).

Specific examples of a preferred cationized cellulose include: polymers obtained by adding glycidyltrimethyl ammonium chloride to hydroxycellulose (Polyquaternium-10, for example, Leoguard G and Leoguard GP (both of which are manufactured by Lion Corporation); and Polymer JR-125, Polymer JR-400, Polymer JR-30M, Polymer LR-400, and Polymer LR-30M (all of which are manufactured by Amerchol Corp.)); and hydroxyethyl cellulose dimethyldiallyl ammonium chloride (Polyquaternium-4, for example, CELLCOAT H-100 and CELLCOAT L-200 (both of which are manufactured by Akzo Nobel)).

The content of the cationic polymer in the agent for hair deforming treatment of the present invention is preferably 0.001 mass% or more, more preferably 0.01 mass% or more, and even more preferably 0.05 mass% or more, and it is preferably 20 mass% or less, and more preferably 10 mass% or less. When the agent for hair deforming treatment is a multi-part agent, the multi-part agent may comprise the cationic polymer in the first agent, or in the second agent, or both of the first and second agents.

Further, the agent for hair deforming treatment of the present invention may also comprise an antioxidant in an amount ordinarily comprised in such an agent. The antioxidant may be an antioxidant generally used in the field of hair cosmetics, and an example of the antioxidant is ascorbic acid.

The agent for hair deforming treatment of the present invention may comprise, as appropriate, components generally mixed into hair cosmetics, as well as the aforementioned components. However, preferably, the agent for hair deforming treatment of the present invention substantially does not comprise a precursor, which is used in an oxidative hair color for coloring hair by an oxidation reaction between a precursor and a coupler. Specifically, the agent for hair deforming treatment of the present invention substantially does not comprise an aromatic compound having at least one amino group, having another amino group or a hydroxy group at the ortho position or para position of the one amino group, and also having a closed-shell quinoid structure when oxidized. The compound as a component (B) is a compound having a structure similar to resorcin, which is a representative compound as a coupler used in an oxidative hair color. The present invention is characterized in that the component (A) is polymerized with the component (B) in hair, so that it makes possible to freely deform the shape of hair by the subsequent heating, and thus, the present invention is a technology completely different from the use of resorcin in an oxidative hair color.

In addition, the agent for hair deforming treatment of the present invention is also different from the technology disclosed in Patent Document 3, in which an oligomer of glyceraldehyde and resorcin is formed using boric acid or silicic acid, and thus, preferably, the agent for hair deforming treatment of the present invention substantially comprises neither boric acid nor silicic acid.

Moreover, preferably, the agent for hair deforming treatment of the present invention substantially does not comprise a hair reducing agent. The present invention is characterized in that it makes possible to deform hair, not depending on the cleavage of the S-S bond of proteins in hair. Thus, the present invention is a technology completely different from a permanent wave agent for deforming hair by cleaving the S-S bond in hair, using a reducing agent. Examples of the hair reducing agent include thioglycolic acid, dithioglycolic acid, cysteine, acetylcysteine, thiol such as butyrolactonethiol, hydrogen sulfite, and a salt thereof.

In the present invention, the expression "substantially does not comprise" is used to mean that the content of a target compound in the agent for hair deforming treatment is preferably less than 0.1 mass%, more preferably less than 0.01 mass%, and even more preferably, it means that the agent for hair deforming treatment does not comprise a target compound.

Since the agent for hair deforming treatment of the present invention is highly safe to human bodies and has less damage to hair, it can be preferably used particularly for human hair.

### [Method of hair deforming treatment]

A hair treatment for semi-permanently or permanently deforming the shape of hair using the agent for hair deforming treatment of the present invention can be carried out by a method of hair treatment comprising the following steps (i) and (ii):
(i) applying the agent for hair deforming treatment of the present invention to hair, and then allowing the agent to penetrate into it, and
(ii) heating and shaping the hair into which the hair cosmetics has penetrated.

In the step (i), the agent for hair deforming treatment may be applied to dry hair or may also be applied to wet hair. In order to swell hair and to promote penetration of the hair treatment agent into the hair, hair is preferably wetted with water before the step (i). The amount of the agent for hair deforming treatment applied to hair in the step (i) is, at a bath ratio to the mass of the hair (the mass of the agent for hair deforming treatment / the mass of the hair), preferably 0.05 or more, more preferably 0.1 or more, even more preferably 0.25 or more, and further preferably 0.5 or more, and it is preferably 5 or less, more preferably 3 or less, and even more preferably 2 or less. The hair to be treated with the agent for hair deforming treatment may be either the entire hair or a part thereof.

In the step (i), the agent for hair deforming treatment can be applied to hair by any given method. In addition, when the agent for hair deforming treatment is a multi-part agent, the first agent comprising the component (B) may be mixed with the second agent comprising the component (A), and the obtained mixture may be then applied to hair. Otherwise, one of the first agent and the second agent may be applied to hair, and the other part may be then applied onto the portion applied. In a case where one part is applied onto the other part successively, from the viewpoint of promoting penetration of the agent for hair deforming treatment into hair and enhancing the effects, after one part had been applied to hair, the hair, to which the hair treatment agent has been applied, may be allowed to stand, and thereafter, the other part may be applied onto the previously applied part. In such a case, in order to allow the agent for hair deforming treatment to penetrate and diffuse into hair, the standing time is preferably 1 minute or more, more preferably 3 minutes or more, and even more preferably 5 minutes or more, and it is preferably 1 hour or less, more preferably 30 minutes or less, and even more preferably 20 minutes or less. At this time, from the viewpoint of promoting penetration of the agent for hair deforming treatment into hair, heating may be carried out. When such heating is carried out, the heating is preferably carried out at a temperature of from 40°C to 90°C. Moreover, when the two parts are successively applied to hair, the order of applying the parts is not particularly limited. From the viewpoint of promoting penetration of the agent for hair deforming treatment into hair and enhancing the effects, it is more preferable that the second agent be applied to hair, after the first agent has been applied thereto.

Moreover, in the step (i), when the first agent and the second agent are successively applied to hair, after the first agent has been applied and left, a step of rinsing the first agent (hereinafter referred to as an "intermediate rinsing step") may be comprised before the application of the second agent. From the viewpoint of a reduction in the treatment time, it is preferable not to comprise such an intermediate rinsing step. When the intermediate rinsing step is not comprised in the step (i), the molecular weight of the component (B) comprised in the first agent is preferably from 100 to 180, and more preferably from 100 to 140, from the viewpoint of further enhancing the hair deforming effect. On the other hand, from the viewpoint of the improvement of touch feeling after hair deformation treatment, it is preferable to comprise the intermediate rinsing step. When the intermediate rinsing step is comprised in the step (i), the molecular weight of the component (B) comprised in the first agent is preferably from 140 to 1,000, and more preferably from 180 to 1,000, from the viewpoint of further enhancing the hair deforming effect and improving the touch feeling after hair deformation.

Furthermore, when the two parts are successively applied to hair, the amounts of the first agent and the second agent applied are not particularly limited. The two parts are applied, such that the molar ratio of the amount of the component (B) in the first agent applied to hair to the amount of the component (A) in the second agent applied to hair, (B)/(A), can be preferably 0.1 or more, more preferably 0.3 or more, even more preferably 0.5 or more, and further preferably 0.7 or more, and it can be preferably 5 or less, more preferably 2.5 or less, even more preferably 2 or less, further preferably 1.5 or less, and still further preferably 1.2 or less. Specifically, for example, the ratio of the amount of the component (B) applied to hair, which is calculated from the content of the component (B) in the first agent and the amount of the first agent applied onto the hair, and the amount of the component (A) applied to hair, which is calculated from the content of the component (A) in the second agent and the amount of the second agent applied onto the hair, may be within the aforementioned range at a molar ratio.

A step of leaving hair, to which the hair treatment agent has been applied, may be inserted between the step (i) and the step (ii). In such a case, in order to allow the agent for hair deforming treatment to penetrate and diffuse into hair, the standing time is preferably 1 minute or more, more preferably 3 minutes or more, and even more preferably 5 minutes or more, and it is preferably 1 hour or less, more preferably 30 minutes or less, and even more preferably 20 minutes or less.

Moreover, from the viewpoint of promoting penetration of the agent for hair deforming treatment into hair, in the step of leaving hair, the hair may be heated. When the hair is heated, it is preferable to heat it at a temperature of from 40°C to 90°C. By this heating operation, since a low-order oligomer can be produced in hair before the step (ii), heating is preferable, also in that the step (ii) can be carried out more advantageously.

Hair may be rinsed or may not be rinsed between the step (i) and the step (ii). From the viewpoint of sufficiently retaining the components of the hair treatment agent in hair, giving a semi-permanent shape to the hair, and further enhancing the effect of semi-permanently deforming the shape of the hair again by heating, it is preferable not to rinse the hair.

In order to increase interaction between the components (A) and (B) and hair proteins in hair, and in order to promote a condensation reaction between the component (A) and the component (B) in hair to obtain the effects of the present invention, the heating temperature in the step (ii) is preferably 50°C or higher, more preferably 60°C or higher, and even more preferably 80°C or higher, and in order to suppress rapid evaporation of water during heating, the heating temperature is preferably 250°C or lower, more preferably 240°C or lower, and even more preferably 230°C or lower. Examples of the heating method include methods using a hair iron, an electric heating rod, a hot curler, etc.

The heating time applied in the step (ii) is selected, as appropriate, depending on the heating device and/or the heating temperature used. From the viewpoint of allowing the agent for hair deforming treatment to penetrate and diffuse into hair and to promote sufficient polymerization, the heating time is 1 second or more, preferably 5 seconds or more, more preferably 1 minute or more, even more preferably 5 minutes or more, further preferably 15 minutes or more, and still further preferably 30 minutes or more, and in order to suppress hair damage, it is preferably 2 hours or less, more preferably 1 hour or less, and even more preferably 45 minutes or less.

Giving of a hair shape in the step (ii) includes both the giving of a straight shape and the giving of a curly shape. Examples of the method of giving a straight shape to hair include a method of blow-drying hair while pulling the hair with a hand, or a tool such as a comb or a brush, and a method of heating hair using a hair iron. From the viewpoint of the ease of deformation, the method of using a hair iron is preferable. In order to provide a straight shape to hair, while heating the hair using a hair iron, a method of holding hair with a flat iron and then moving the flat iron from the roots to the tips, or a method of holding hair with a flat iron and then retaining it as is, while pulling the hair with a hand, or a tool such as a comb or a brush, may be applied. Otherwise, a combination of the two above methods may also be applied. When a curly shape is given to hair, a method of winding hair around an electric heating rod, a hot curler, etc., and then retaining it as is, while heating the hair, a method of winding hair around a curl iron and then retaining it as is, and the like are applied.

The step (ii) is preferably carried out in an environment in which evaporation of water is suppressed. Examples of a specific means for suppressing evaporation of water include a method of coating hair, to which the hair treatment agent has been applied, with a film-like substance made from a material, through which water vapor cannot penetrate, a cap, etc., and a method of continuously spraying water vapor such as superheated vapor to hair.

After completion of the step (ii), hair may be rinsed, or may not be rinsed. From the viewpoint of preventing a reduction in touch feeling of the hair due to redundant polymers, the hair is preferably rinsed.

It is considered that, by these treatments, the components (A) and (B) penetrate into hair, and thereby, an interaction of these components with hair proteins occurs. In addition, in the hair, a thermoplastic condensate of the components (A) and (B) is generated. Thus, the shape of hair can be easily deformed by heating hair, and further, once the treatment is performed, hair can be repeatedly deformed semi-permanently or permanently only by heating the hair, without applying the hair treatment agent again. Moreover, the hair deformation given by the method of the present invention is not lost even by washing it with shampoo or the like.

### (Re-deforming treatment method)

After hair has been subjected to deforming treatment by a method comprising the step (i) or the step (ii), semi-permanently re-deforming the hair to another shape by heating it can be carried out. When hair is re-deformed, it is preferable to heat the hair at a temperature of preferably 30°C or higher, and more preferably 40°C or higher, and at a temperature of preferably 230°C or lower, more preferably 220°C or lower, and even more preferably 210°C or lower. In addition, when hair is subjected to re-deforming treatment, it is preferable not to apply any one of the agent for hair deforming treatment of the present invention, a hair treatment agent comprising a reducing agent, such as, what is called, a permanent wave agent, and known agent for hair deforming treatments such as an alkaline relaxer.

Hereafter, specific procedures for carrying out a step of semi-permanently re-deforming hair to another shape by heating it will be described.

### • Case of re-deforming hair subjected to deforming treatment into curly shape into straight shape

In order to re-deform hair, which has been once subjected to deforming treatment into a curly shape, into a straight shape, a method of blow-drying hair with a dryer, while pulling the hair with a hand, or a tool such as a comb or a brush, a method of heating hair with a hair iron, and the like are applied. From the viewpoint of the ease of deformation, the method of using the hair iron is preferable. In order to provide a straight shape to hair, while heating the hair with a hair iron, a method of holding hair with a hair iron and then moving the hair iron from the roots to the tips, or a method of holding hair with a hair iron and then retaining it as is, while pulling the hair with a hand, or a tool such as a comb or a brush, may be applied. Otherwise, a combination of the two above methods may also be applied.

In this case, from the viewpoint of deforming the shape of hair semi-permanently or permanently, regardless of the type of a hair iron used, the material of a heating portion, a preset temperature, and the operational method of a hair iron, the attained temperature during the heating of hair (the temperature of hair) is preferably 120°C or higher, and more preferably 150°C or higher, and also, from the viewpoint of achieving both prevention of hair damage and semi-permanent or permanent deformation of the shape of hair, the attained temperature is preferably 230°C or lower, more preferably 220°C or lower, and even more preferably 210°C or lower. The temperature at which hair is heated can be measured, for example, using a radiation thermometer (Model No.: ST653) manufactured by SENTRY.

### • Case of re-deforming hair subjected to deforming treatment into straight shape into curly shape

In order to re-deform hair, which has been once subjected to deforming treatment into a straight shape, into a curly shape, a method of winding hair around a rod, a curler, etc., and then retaining it as is, while heating the hair, a method of winding hair around a hair iron and then retaining it as is, and the like are applied.

In this case, from the viewpoint of deforming the shape of hair semi-permanently or permanently, the attained temperature during the heating of hair (the temperature of hair) is preferably 30°C or higher, and more preferably 40°C or higher, and from the viewpoint of achieving both prevention of hair damage and semi-permanent or permanent deformation of the shape of hair, the attained temperature is preferably 180°C or lower, more preferably 120°C or lower, even more preferably 100°C or lower, further preferably 80°C or lower, and still further preferably 60°C or lower.

Even in a case where hair is re-deformed, upon heating it, either a method of heating hair, which is dried, or a method of heating hair after wetting it with water, may be applied. From the viewpoint of semi-permanently or permanently deforming the shape of hair, the method of heating hair after wetting it with water is preferable.

The heating time applied when hair is re-deformed is selected, as appropriate, depending on a heating device used and a heating temperature. From the viewpoint of semi-permanently or permanently deforming the shape of hair, the heating time is preferably 1 second or more, more preferably 5 seconds or more, even more preferably 1 minute or more, further preferably 5 minutes or more, still further preferably 15 minutes or more, and still further preferably 30 minutes or more. In addition, in order to suppress hair damage, it is preferably 2 hours or less, more preferably 1 hour or less, and even more preferably 45 minutes or less.

Preferred method of hair treatments, which semi-permanently or permanently deform hair, include the following three patterns.

### Pattern 1: Case where the hair treatment agent is one-part agent

1) Hair is optionally wetted with water.
2) The hair treatment agent of the present invention, which comprises the following components (A), (B) and (C) wherein the molar ratio of the content of the component (B) to the content of the component (A) is 0.1 to 5, is applied to hair, and it is then allowed to penetrate into it:
   Component (A): glyoxylic acid or a hydrate or salt thereof,
   Component (B): a compound represented by the formula (1), and
   Component (C): water.
3) The hair, to which the hair treatment agent has been applied, is optionally allowed to stand for 1 minute or more and 1 hour or less. During this operation, the hair is optionally heated to a temperature of from 40°C to 90°C.
4) The hair is heated and shaped at a temperature of from 50°C to 250°C.
5) The hair is optionally rinsed.
6) The hair is optionally heated at a temperature of from 40°C to 230°C and re-deformed.

### Pattern 2: Case where the hair treatment agent is two-part agent

1) Hair is optionally wetted with water.
2) The hair treatment agent of the present invention, in which a first agent comprising the following components (B) and (C) is mixed with a second agent comprising the following components (A) and (C) so that the molar ratio of the content of the component (B) to the content of the component (A) can be 0.1 to 5, is applied to hair, and it is then allowed to penetrate into it:
   Component (A): glyoxylic acid or a hydrate or salt thereof,
   Component (B): a compound represented by the formula (1), and,
   Component (C): water.
3) The hair, to which the hair treatment agent has been applied, is optionally allowed to stand for 1 minute or more and 1 hour or less. During this operation, the hair is optionally heated to a temperature of from 40°C to 90°C.
4) The hair is heated and shaped at a temperature of from 50°C to 250°C.
5) The hair is optionally rinsed.
6) The hair is optionally heated at a temperature of from 40°C to 230°C and re-deformed.

### Pattern 3: Case where the hair treatment agent is two-part agent

1) Hair is optionally wetted with water.
2) A first agent comprising the following components (B) and (C) is applied to hair, and it is then allowed to penetrate into it:
   Component (B): a compound represented by the formula (1), and
   Component (C): water.
3) The hair is optionally allowed to stand for 1 minute or more and 1 hour or less. During this operation, the hair is optionally heated to a temperature of from 40°C to 90°C.
4) The first agent is optionally washed away from the hair.
5) The second agent comprising the following components (A) and (C) is applied onto the portion in hair, to which the first agent has been applied, so that the molar ratio of the content of the component (B) to the content of the component (A) can be 0.1 to 5, and it is then allowed to penetrate into the hair:
   Component (A): glyoxylic acid or a hydrate or salt thereof, and
   Component (C): water.
6) The hair is optionally allowed to stand for 1 minute or more and 1 hour or less. During this operation, the hair is optionally heated to a temperature of from 40°C to 90°C.
7) The hair is heated and shaped at a temperature of from 50°C to 250°C.
8) The hair is optionally rinsed.
9) The hair is optionally heated at a temperature of from 40°C to 230°C, to re-deform the hair.

Since the method of hair treatment of the present invention is a technology capable of freely changing hair based on a principle, which is completely different from a permanent wave treatment using a reducing agent or a relaxer treatment using a strongly-alkaline hair treatment agent, the present method does not comprise a step of applying a hair treatment agent comprising a reducing agent or a strongly-alkaline hair treatment agent to the hair. Accordingly, it can be said that, in comparison to the aforementioned conventional hair deformation methods, the method of hair treatment of the present invention is also advantageous in that hair can be deformed without being damaged.

With regard to the aforementioned embodiments, preferred aspects of the present invention will be further disclosed below.
<1> An agent for hair deforming treatment comprising the following components (A), (B) and (C):
   (A) glyoxylic acid or a hydrate or salt thereof,
   (B) a compound represented by the following formula (1). 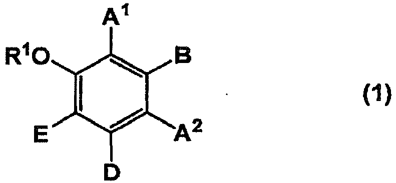 wherein
      R¹ represents a hydrogen atom or a methyl group,
      A¹ and A², which may be the same or different, each represent a hydrogen atom, a straight-chain or branched-chain alkyl group or alkenyl group containing from 1 to 12 carbon atoms, an optionally substituted aralkyl group or arylalkenyl group containing from 7 to 12 carbon atoms, a straight-chain or branched-chain alkoxy group or alkenyloxy group containing from 1 to 6 carbon atoms, a halogen atom, or -CO-R² (wherein R² represents a straight-chain or branched-chain alkyl group or alkenyl group containing from 1 to 12 carbon atoms, an optionally substituted aralkyl group or arylalkenyl group containing from 7 to 12 carbon atoms, or an optionally substituted aromatic hydrocarbon group containing from 6 to 12 carbon atoms),
      B represents a hydrogen atom, a straight-chain or branched-chain alkyl group or alkenyl group containing from 1 to 12 carbon atoms, an optionally substituted aralkyl group or arylalkenyl group containing from 7 to 12 carbon atoms, -OR³, or -COOR³ (wherein R³ represents a hydrogen atom, or a straight-chain or branched-chain alkyl group or alkenyl group containing from 1 to 6 carbon atoms),
      D represents a hydrogen atom, a hydroxy group, a methyl group, or a straight-chain or branched-chain alkoxy group or alkenyloxy group containing from 1 to 12 carbon atoms, and
      E represents a hydrogen atom, a hydroxy group, a straight-chain or branched-chain alkyl group or alkenyl group containing from 1 to 6 carbon atoms, or a straight-chain or branched-chain alkoxy group or alkenyloxy group containing from 1 to 6 carbon atoms,
      provided that two or three of A¹, A², B and E are hydrogen atoms, and the remaining groups do not include sulfo groups, and that when D is a hydrogen atom or a methyl group, A¹ and B, or A² and B, together with two carbon atoms adjacent to them, form a benzene ring optionally substituted with a hydroxy group.
   And, (C) water.
<2> The agent for hair deforming treatment according to <1>, wherein the molar ratio of the content of the component (B) to the content of the component (A), (B)/(A), is preferably 0.1 or more, more preferably 0.3 or more, even more preferably 0.4 or more, further preferably 0.5 or more, and still further preferably 0.7 or more, and it is preferably 5 or less, more preferably 2.5 or less, even more preferably 2 or less, further preferably 1.5 or less, and still further preferably 1.2 or less.
<3> The agent for hair deforming treatment according to <1> or <2>, wherein the content of the component (A) is, in terms of glyoxylic acid, preferably 1 mass% or more, more preferably 2 mass% or more, even more preferably 2.5 mass% or more, and further preferably 3 mass% or more, and it is preferably 30 mass% or less, more preferably 25 mass% or less, even more preferably 20 mass% or less, further preferably 15 mass% or less, and still further preferably 12 mass% or less.
<4> The agent for hair deforming treatment according to any one of <1> to <3>, wherein the content of the component (B) is preferably 0.2 mass% or more, more preferably 0.5 mass% or more, even more preferably 1 mass% or more, further preferably 5 mass% or more, and still further preferably 10 mass% or more, and it is preferably 30 mass% or less, more preferably 25 mass% or less, even more preferably 23 mass% or less, and further preferably 20 mass% or less.
<5> The agent for hair deforming treatment according to any one of <1> to <4>, wherein the component (B) is preferably one or two or more selected from the group consisting of compounds represented by the following formulae (1-1), (1-2), and (1-3-a) or (1-3-b): 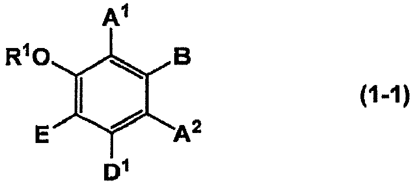 wherein R¹, A¹, A², B and E are as defined above, and D¹ represents a hydroxy group or a methoxy group, 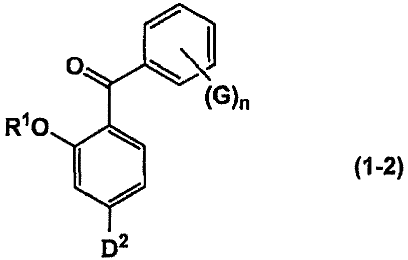 wherein R¹ is as defined above, D² represents a hydroxy group or an alkoxy group containing from 1 to 12 carbon atoms, G represents a hydroxy group, a straight-chain or branched-chain alkyl group or alkenyl group containing from 1 to 6 carbon atoms, or an alkoxy group containing from 1 to 6 carbon atoms, and n represents an integer of from 0 to 2, and 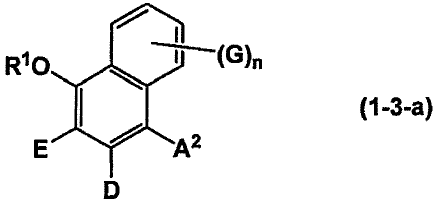 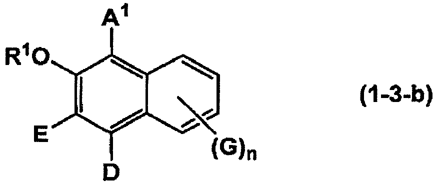 wherein R¹, A¹, E, D, G and n are as defined above.
<6> The agent for hair deforming treatment according to <5>, wherein the compound represented by the formula (1-1) is preferably an m-dimethoxybenzene derivative represented by the following formula (1-1-1): 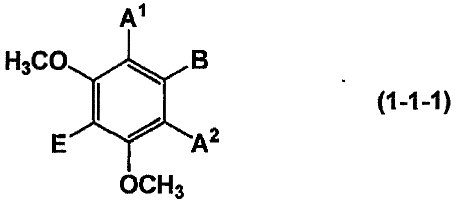 wherein A¹, A², B and E are as defined above.
<7> The agent for hair deforming treatment according to <6>, wherein A¹ and A² each represent, preferably a hydrogen atom or a straight-chain or branched-chain alkyl group or alkenyl group containing from 1 to 4 carbon atoms, and more preferably a hydrogen atom.
<8> The agent for hair deforming treatment according to <6> or <7>, wherein B represents: preferably a hydrogen atom, a straight-chain or branched-chain alkyl group or alkenyl group containing from 1 to 12 carbon atoms, an optionally substituted aralkyl group or arylalkenyl group containing from 7 to 12 carbon atoms, or -OR³ (wherein R³ represents a hydrogen atom, or a straight-chain or branched-chain alkyl group or alkenyl group containing from 1 to 6 carbon atoms); more preferably a hydrogen atom, an alkyl group or alkenyl group containing from 1 to 4 carbon atoms, an optionally substituted arylalkenyl group containing from 7 to 10 carbon atoms, or a hydroxy group; and even more preferably a hydrogen atom, an optionally substituted arylalkenyl group containing from 7 to 10 carbon atoms, or a hydroxy group.
<9> The agent for hair deforming treatment according to any one of <6> to <8>, wherein E represents, preferably a hydrogen atom, or a straight-chain or branched-chain alkyl group or alkenyl group containing from 1 to 4 carbon atoms, and more preferably a hydrogen atom.
<10> The agent for hair deforming treatment according to <5>, wherein the compound represented by the formula (1-1) is preferably an m-methoxyphenol derivative represented by the following formula (1-1-2): 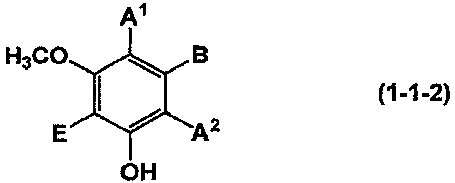 wherein A¹, A², B and E are as defined above.
<11> The agent for hair deforming treatment according to <10>, wherein A¹ and A² each represent: preferably a hydrogen atom, a straight-chain or branched-chain alkyl group or alkenyl group containing from 1 to 12 carbon atoms, or an optionally substituted aralkyl group or arylalkenyl group containing from 7 to 12 carbon atoms; and more preferably a hydrogen atom, a straight-chain or branched-chain alkyl group containing from 1 to 6 carbon atoms, or an optionally substituted arylalkenyl group containing from 7 to 10 carbon atoms.
<12> The agent for hair deforming treatment according to <10> or <11>, wherein B represents: preferably a hydrogen atom, a straight-chain or branched-chain alkyl group or alkenyl group containing from 1 to 12 carbon atoms, an optionally substituted aralkyl group or arylalkenyl group containing from 7 to 12 carbon atoms, or -OR³ (wherein R³ represents a hydrogen atom, or a straight-chain or branched-chain alkyl group or alkenyl group containing from 1 to 6 carbon atoms); more preferably a hydrogen atom, an alkyl group or alkenyl group containing from 1 to 4 carbon atoms, an optionally substituted arylalkenyl group containing from 7 to 10 carbon atoms, or a hydroxy group; and even more preferably a hydrogen atom, an optionally substituted arylalkenyl group containing from 7 to 10 carbon atoms, or a hydroxy group.
<13> The agent for hair deforming treatment according to any one of <10> to <12>, wherein E represents: preferably a hydrogen atom, a hydroxy group, a straight-chain or branched-chain alkyl group or alkenyl group containing from 1 to 4 carbon atoms, or a straight-chain or branched-chain alkoxy group or alkenyloxy group containing from 1 to 4 carbon atoms; and more preferably a hydrogen atom or a hydroxy group.
<14> The agent for hair deforming treatment according to <5>, wherein the compound represented by the formula (1-1) is preferably a resorcin derivative represented by the following formula (1-1-3): 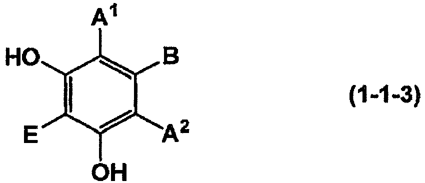 wherein A¹, A², B and E are as defined above.
<15> The agent for hair deforming treatment according to <14>, wherein the resorcin derivative represented by the formula (1-1-3) is preferably a resorcin derivative represented by the following formula (i): 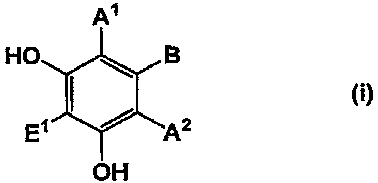 wherein A¹, A² and B are as defined above, and E¹ represents a hydroxy group, a straight-chain or branched-chain alkyl group or alkenyl group containing from 1 to 6 carbon atoms, or a straight-chain or branched-chain alkoxy group or alkenyloxy group containing from 1 to 6 carbon atoms.
<16> The agent for hair deforming treatment according to <15>, wherein A¹ and A² each represent: preferably a hydrogen atom, or a straight-chain or branched-chain alkyl group or alkenyl group containing from 1 to 12 carbon atoms; and more preferably a hydrogen atom.
<17> The agent for hair deforming treatment according to <15> or <16>, wherein B preferably represents a hydrogen atom, an optionally substituted aralkyl group or arylalkenyl group containing from 7 to 12 carbon atoms, or -OR³ (wherein R³ represents a hydrogen atom, or a straight-chain or branched-chain alkyl group or alkenyl group containing from 1 to 4 carbon atoms).
<18> The agent for hair deforming treatment according to any one of <15> to <17>, wherein E¹ preferably represents a hydroxy group, a straight-chain or branched-chain alkyl group or alkenyl group containing from 1 to 4 carbon atoms, or a straight-chain or branched-chain alkoxy group or alkenyloxy group containing from 1 to 4 carbon atoms.
<19> The agent for hair deforming treatment according to any one of <15> to <18>, wherein the resorcin derivative represented by the formula (i) is preferably 2-alkyl resorcin, pyrogallol, 2-methoxy resorcin, gallic acid or gallic acid ester, and more preferably 2-alkyl resorcin, gallic acid or gallic acid ester.
<20> The agent for hair deforming treatment according to <14>, wherein the resorcin derivative represented by the formula (1-1-3) is preferably a resorcin derivative represented by the following (ii-1) : 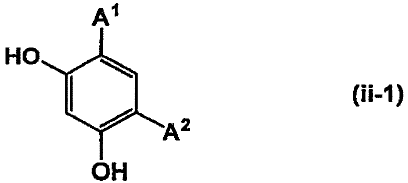 wherein A¹ and A² are as defined above.
<21> The agent for hair deforming treatment according to <20>, wherein the resorcin derivative represented by the formula (ii-1) represents: preferably 4-alkyl resorcin, 4-alkenyl resorcin, 4-aralkyl resorcin, 4-hydroxyaralkyl resorcin, 4-arylalkenyl resorcin, 4-hydroxyarylalkenyl resorcin, 4-(1-methylnaphthyl) resorcin, 4-alkoxy resorcin, halogenated resorcin, 4-alkanoyl resorcin, or 4-arylalkanoyl resorcin; more preferably 4-alkyl resorcin, 4-aralkyl resorcin, halogenated resorcin, 4-alkanoyl resorcin, or 4-arylalkanoyl resorcin; and even more preferably 4-alkyl resorcin or 4-aralkyl resorcin.
<22> The agent for hair deforming treatment according to <14>, wherein the resorcin derivative represented by the formula (1-1-3) is preferably a resorcin derivative represented by the following formula (ii-2): 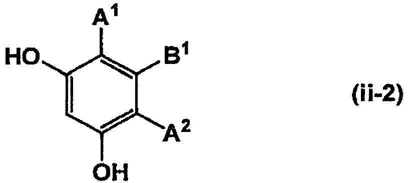 wherein A¹ and A² are as defined above, and B¹ represents a straight-chain or branched-chain alkyl group or alkenyl group containing from 1 to 12 carbon atoms, an optionally substituted aralkyl group or arylalkenyl group containing from 7 to 12 carbon atoms, -OR³, or -COOR³ (wherein R³ represents a hydrogen atom, or a straight-chain or branched-chain alkyl group or alkenyl group containing from 1 to 6 carbon atoms.
<23> The agent for hair deforming treatment according to <22>, wherein the resorcin derivative represented by the formula (ii-2) is preferably a resorcin derivative represented by the following formula (ii-2-a): 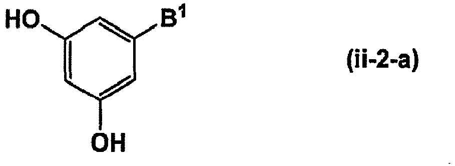 wherein B¹ is as defined above.
<24> The agent for hair deforming treatment according to <23>, wherein the resorcin derivative represented by the formula (ii-2-a) represents: preferably 5-alkyl resorcin, 5-alkenyl resorcin, phloroglucinol, 5-alkoxybenzene-1,3-diol, 3,5-dihydroxybenzoic acid, 3,5-dihydroxybenzoic acid ester, 5-aralkyl resorcin, 5-hydroxyaralkyl resorcin, 5-arylalkenyl resorcin, or 5-hydroxyarylalkenyl resorcin; and more preferably 5-alkyl resorcin, 5-aralkyl resorcin, 5-hydroxyaralkyl resorcin, or 5-hydroxyarylalkenyl resorcin.
<25> The agent for hair deforming treatment according to <22>, wherein the resorcin derivative represented by the formula (ii-2) is preferably a resorcin derivative represented by the following formula (ii-2-b): 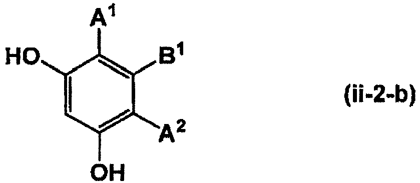 wherein A¹, A² and B¹ are as defined above.
<26> The agent for hair deforming treatment according to <25>, wherein A¹ and A² each represent: preferably a hydrogen atom, a straight-chain or branched-chain alkyl group or alkenyl group containing from 1 to 4 carbon atoms, or an alkoxy group or alkenyloxy group containing from 1 to 4 carbon atoms.
<27> The agent for hair deforming treatment according to <26>, wherein the resorcin derivative represented by the formula (ii-2-b) represents: preferably 2-alkylbenzene-1,3,5-triol, phloroglucin acid ester, or 3,5-dihydroxybenzoic acid ester; and more preferably phloroglucin acid ester.
<28> The agent for hair deforming treatment according to <5>, wherein the benzophenone derivative represented by the formula (1-2) represents: preferably benzophenone-1, benzophenone-2, benzophenone-3, benzophenone-6, benzophenone-8, benzophenone-10, or benzophenone-12; and more preferably benzophenone-12.
<29> The agent for hair deforming treatment according to <5>, wherein the naphthol derivative represented by the formula (1-3-a) or (1-3-b) is a naphthol derivative, wherein, in the formula (1-3-a) or (1-3-b), R¹ represents preferably a hydrogen atom, or an alkyl group or alkenyl containing from 1 to 4 carbon atoms, and more preferably a hydrogen atom.
<30> The agent for hair deforming treatment according to <29>, wherein the naphthol derivative represented by the formula (1-3-a) or (1-3-b) is a naphthol derivative, wherein, in the formula (1-3-a) or (1-3-b), A¹ and A² each represent, preferably a hydrogen atom, a hydroxy group, a straight-chain or branched-chain alkyl group containing from 1 to 4 carbon atoms, or an alkoxy group containing from 1 to 4 carbon atoms, and more preferably a hydrogen atom or a hydroxy group.
<31> The agent for hair deforming treatment according to <29> or <30>, wherein the naphthol derivative represented by the formula (1-3-a) or (1-3-b) is a naphthol derivative, wherein, in the formula (1-3-a) or (1-3-b), D preferably represents a hydrogen atom, a hydroxy group, a straight-chain or branched-chain alkyl group containing from 1 to 4 carbon atoms, or an alkoxy group containing from 1 to 4 carbon atoms.
<32> The agent for hair deforming treatment according to any one of <29> to <31>, wherein the naphthol derivative represented by the formula (1-3-a) or (1-3-b) is a naphthol derivative, wherein, in the formula (1-3-a) or (1-3-b), E preferably represents a hydrogen atom, a hydroxy group, or an alkyl group containing from 1 to 4 carbon atoms or an alkoxy group containing from 1 to 4 carbon atoms.
<33> The agent for hair deforming treatment according to any one of <29> to <32>, wherein the naphthol derivative represented by the formula (1-3-a) or (1-3-b) is 1-naphthol, 2-naphthol, 3-methylnaphthalene-1-ol, naphthalene-1,4-diol, naphthalene-1,5-diol, or naphthalene-1,8-diol.
<34> The agent for hair deforming treatment according to any one of <1> to <33>, wherein the component (B) is: preferably one or two or more selected from the group consisting of the m-dimethoxybenzene derivative represented by the formula (1-1-1), the resorcin derivative represented by the formula (1-1-3), the benzophenone derivative represented by the formula (1-2), and the naphthol derivative represented by the formula (1-3-a) or (1-3-b); more preferably one or two or more selected from the group consisting of 2-methyl resorcin, 4-chloro resorcin, 4-alkyl resorcin, 4-aralkyl resorcin, 4-acylated resorcin, 5-alkyl resorcin, 5-aralkyl resorcin, 5-hydroxyarylalkenyl resorcin, phloroglucin acid ester, gallic acid, and gallic acid ester; even more preferably one or two or more selected from the group consisting of 4-butyl resorcin (trivial name: Rucinol), 4-(1-phenylethyl) resorcin (trivial name: Symwhite 377), 5-(hydroxyphenylethenyl) resorcin (trivial name: resveratrol), 3-hydroxyphenyl-1-(benzene-2,4,6-triol)propan-1-one (trivial name: Phloretin), 4-(2,4-dihydroxybenzoyl) resorcin (trivial name: Benzophenone-2), 5-(hydroxyphenylethenyl)-1,3-dimethoxybenzene (trivial name: Pterostilbene), and 1-naphthol; and further preferably one or two or more selected from the group consisting of 2-methyl resorcin, 4-chloro resorcin, 1-naphthol, 4-n-butyl resorcinol, 4-phenyl resorcinol, 5-(hydroxyphenylethenyl) resorcin, 3-hydroxyphenyl-1-(benzene-2,4,6-triol)propan-1-one, and 4-(2,4-dihydroxybenzoyl) resorcin.
<35> The agent for hair deforming treatment according to any one of <1> to <34>, wherein the molecular weight of the component (B) is preferably 120 or more, and from the viewpoint of permeability into hair, it is preferably 1,000 or less, more preferably 500 or less, and even more preferably 300 or less.
<36> The agent for hair deforming treatment according to any one of <1> to <35>, which comprises an aromatic compound having at least one amino group, having another amino group or a hydroxy group at the ortho positon or the para position of the one amino group, and also having a closed-shell quinoid structure when oxidized, in an amount of preferably less than 0.1 mass%, and more preferably less than 0.01 mass%, and even more preferably, the agent for hair deforming treatment does not comprise the compound therein.
<37> The agent for hair deforming treatment according to any one of <1> to <36>, wherein, preferably, deformation of hair is not caused by the cleavage and recombination of the S-S bond of hair proteins.
<38> The agent for hair deforming treatment according to any one of <1> to <37>, wherein the total amount of components for reducing proteins in hair is preferably less than 0.1 mass%, and more preferably less than 0.01 mass%, and even more preferably, the agent for hair deforming treatment does not comprise the compounds therein.
<39> The agent for hair deforming treatment according to <38>, wherein the components for reducing proteins in hair are thiol, hydrogen sulfite, and salt thereof.
<40> The agent for hair deforming treatment according to <39>, wherein the thiol is preferably thioglycolic acid, dithioglycolic acid, cysteine, acetylcysteine, or butyrolactonethiol.
<41> The agent for hair deforming treatment according to any one of <1> to <40>, wherein the pH thereof is preferably 4 or less, more preferably 3 or less, even more preferably 2.5 or less, and further preferably 2 or less, and it is preferably 1 or more, more preferably 1.2 or more, and more preferably 1.5 or more.
<42> The agent for hair deforming treatment according to any one of <1> to <41>, which is preferably a multi-part agent comprising a first agent comprising the component (B) and a second agent comprising the component (A).
<43> The agent for hair deforming treatment according to <42>, wherein the content of the component (A) in the second agent is, in terms of glyoxylic acid, preferably 1 mass% or more, more preferably 2 mass% or more, even more preferably 2.5 mass% or more, and further preferably 3 mass% or more, and it is preferably 30 mass% or less, more preferably 25 mass% or less, even more preferably 20 mass% or less, further preferably 15 mass% or less, and still further preferably 12 mass% or less.
<44> The agent for hair deforming treatment according to <42> or <43>, wherein the content of the component (B) in the first agent is preferably 0.2 mass% or more, more preferably 0.5 mass% or more, even more preferably 1 mass% or more, further preferably 5 mass% or more, and still further preferably 10 mass% or more, and it is preferably 30 mass% or less, more preferably 25 mass% or less, even more preferably 23 mass% or less, and further preferably 20 mass% or less.
<45> The agent for hair deforming treatment according to any one of <42> to <44>, wherein the pH of the second agent is preferably 4 or less, more preferably 3 or less, even more preferably 2.5 or less, and further preferably 2 or less, and it is preferably 1 or more, more preferably 1.2 or more, and even more preferably 1.5 or more.
<46> The agent for hair deforming treatment according to any one of <42> to <45>, wherein the composition after completion of the mixing of the components is the composition according to any one of <1> to <41>.
<47> A method of hair treatment for semi-permanently or permanently deforming the shape of hair, which comprises the following steps (i) and (ii):
   (i) applying the agent for hair deforming treatment according to any one of <1> to <46> to hair, and then allowing the agent to penetrate into the hair, and
   (ii) heating and shaping the hair into which the agent for hair deforming treatment has penetrated.
<48> The method of hair treatment according to <47>, wherein the step (i) is preferably a step of mixing the first agent of the multi-part agent for hair deforming treatment according to any one of <42> to <46> with the second agent thereof, and then applying the obtained mixture to hair.
<49> The method of hair treatment according to <47>, wherein the step (i) is preferably a step of applying one of the first agent and the second agent in the multi-part agent for hair deforming treatment according to any one of <42> to <46> to hair, and then applying the other part onto the portion applied.
<50> The method of hair treatment according to <49>, wherein the step (i) is preferably a step of applying the first agent comprising the component (B), and then applying the second agent comprising the component (A) onto the portion applied.
<51> The method of hair treatment according to <49> or <50>, wherein the two parts are applied, such that the molar ratio of the amount of the component (B) comprised in the first agent applied to hair to the amount of the component (A) comprised in the second agent applied to hair, (B)/(A), is preferably 0.1 or more, more preferably 0.3 or more, even more preferably 0.5 or more, and further preferably 0.7 or more, and it is preferably 5 or less, more preferably 2.5 or less, even more preferably 2 or less, further preferably 1.5 or less, and still further preferably 1.2 or less.
<52> The method of hair treatment according to any one of <49> to <51>, which preferably comprises a step of leaving hair, to which one of the first agent and the second agent has been applied, between the step of applying the one part to the hair and the step of applying the other part onto the portion applied.
<53> The method of hair treatment according to <52>, wherein when the hair to which one part has been applied is allowed to stand, the hair is allowed to stand, preferably while being heated at a temperature of from 40°C to 90°C.
<54> The method of hair treatment according to any one of <47> to <53>, which preferably comprises a step of wetting hair before the step (i).
<55> The method of hair treatment according to any one of <47> to <54>, wherein the heating temperature in the step (ii) is preferably 50°C or higher, more preferably 60°C or higher, and even more preferably 80°C or higher, and it is preferably 250°C or lower, more preferably 240°C or lower, and even more preferably 230°C or lower.
<56> The method of hair treatment according to any one of <47> to <55>, wherein the step (ii) is preferably carried out under an environment in which evaporation of water is suppressed.
<57> The method of hair treatment according to any one of <47> to <56>, which preferably does not comprise a step of applying a hair treatment agent comprising a reducing agent or a strongly-alkaline hair treatment agent having pH of from 12 to 14 to the hair.
<58> The method of hair treatment according to any one of <47> to <57>, wherein the mass of the agent for hair deforming treatment applied to hair in the step (i) is, at a bath ratio of the mass of the agent for hair deforming treatment to the mass of the hair (the mass of the agent for hair deforming treatment / the mass of the hair), preferably 0.05 or more, more preferably 0.1 or more, even more preferably 0.25 or more, and further preferably 0.5 or more, and it is preferably 5 or less, more preferably 3 or less, and even more preferably 2 or less.
<59> The method of hair treatment according to any one of <47> to <58>, wherein the heating time in the step (ii) is preferably 1 second or more, more preferably 5 seconds or more, even more preferably 1 minute or more, further preferably 5 minutes or more, still further preferably 15 minutes or more, and still further preferably 30 minutes or more, and it is preferably 2 hours or less, more preferably 1 hour or less, and even more preferably 45 minutes or less.
<60> The method of hair treatment according to any one of <47> to <59>, which preferably comprises a step of leaving hair, to which the hair treatment agent has been applied, between the step (i) and the step (ii).
<61> The method of hair treatment according to <60>, wherein the leaving time is preferably 1 minute or more, more preferably 3 minutes or more, and even more preferably 5 minutes or more, and it is preferably 1 hour or less, more preferably 30 minutes or less, and even more preferably 20 minutes or less.
<62> The method of hair treatment according to <60> or <61>, wherein hair is preferably heated at a temperature of from 40°C to 90°C during the leaving time between the step (i) and the step (ii).
<63> The method of hair treatment according to any one of <47> to <62>, which preferably does not comprise a step of rinsing hair, to which the hair treatment agent has been applied, between the step (i) and the step (ii).
<64> The method of hair treatment according to any one of <47> to <63>, which preferably comprises a step of rinsing hair after the step (ii).
<65> The method of hair treatment according to any one of <47> to <64>, wherein hair is preferably re-deformed into a different shape by heating, after the step (ii).
<66> The method of hair treatment according to <65>, wherein preferably the agent for hair deforming treatment is not applied, when hair is re-deformed.
<67> The method of hair treatment according to <65> or <66>, wherein the heating temperature when hair is re-deformed is preferably 30°C or higher, and more preferably 40°C or higher, and it is preferably 230°C or lower, more preferably 220°C or lower, and even more preferably 210°C or lower.
<68> Use of the composition according to any one of <1> to <46> for semi-permanent or permanent hair deformation.

### [Examples]

### Examples 1 to 11 and Comparative Examples 1 to 8

The treatment agents shown in Table 1 were prepared, and the following three-step hair treatment processes were then carried out using the agents. The shape-giving effect of each agent at each step was evaluated. The results are also shown in Table 1. It is to be noted that the pH value of each composition was measured by leaving the prepared composition at a room temperature (25°C) using a pH meter (manufactured by HORIBA / Model No.: F-52), without diluting each composition after the preparation thereof.

### <I: Imparting semi-permanent curly shape>

1. A tress in length of 25 cm, which consisted of 0.5 g of Caucasian straight hair (untreated hair), was wetted with tap water at 30°C for 30 seconds, and the wet tress was then wound around a plastic rod with a diameter of 14 mm, and fixed with a clip.
2. 1 g of the treatment agent was applied to the tress wound around the rod, and the rod was entirely covered with a plastic film for hermetical sealing. The tress was then heated for 1 hour in an oven, which was set at 90°C.
3. The tress was removed from the oven, and cooled to a room temperature.
4. The tress was removed from the rod, and was then rinsed with running tap water at 30°C for 30 seconds. Thereafter, a shampoo for evaluation was lathered on the tress for 60 seconds.
5. The tress was rinsed with running tap water at 30°C for 30 seconds, and immersed at an infinite bath ratio in tap water at 30°C for 60 seconds. Thereafter, the tress was gently pulled up out of the water while the root thereof was held, and water was then drained off by lightly shaking it.
6. The tress was hung and allowed to stand in a laboratory for 2 hours, to be dried. The tress was combed, was then hung, and was then photographed from the side. Based on the photograph, the radius of curvature of the strongest curly portion in the tress was measured. The obtained value was doubled to obtain a curl diameter.

### (Evaluation criteria for the effect of giving initial shape to hair)

A: The curl diameter is 1 time or more and less than 2 times as large as the used rod (diameter: 14 mm)
B: The curl diameter is 2 times or more and less than 3 times as large as the used rod (diameter: 14 mm)
C: The curl diameter is 3 times or more and less than 4 times as large as the used rod (diameter: 14 mm)
D: The curl diameter is 4 times or more and less than 50 times as large as the used rod (diameter: 14 mm)
E: Straight hair was maintained, and the hair shape has not changed from before the treatment.

### <II: Imparting semi-permanent straight shape to semi-permanent curly shaped hair>

1. The tress, which had been evaluated in <I: Imparting semi-permanent curly shape> above, was combed to detangle it, and an iron with an actual temperature of 180°C was slid through the tress at a rate of 5 cm/sec six times.
2. The tress was rinsed with running tap water at 30°C for 30 seconds, and a shampoo for evaluation was lathered on the tress for 60 seconds. Thereafter, the tress was rinsed with running tap water at 30°C for 30 seconds, and it was then dried with a towel.
3. The tress was dried, while being shaken, so that the natural shape of hair could appear (wherein dryer was not used), and it was then combed. Thereafter, the tress was hung, and was then visually observed from the side.

### (Evaluation criteria for the effect of giving shape to hair again)

A: The curl has not remained, and the hair is completely deformed into straight hair.
B: The curl has become weaker than before the treatment with a flat iron, but the hair has not been completely deformed into straight hair.
C: The curly hair remains as is, and the hair shape has not changed from before the treatment.

### <III: Imparting semi-permanent curly shape to semi-permanent straight shaped hair>

1. The tress, which had been evaluated in <II: Imparting semi-permanent straight shape to semi-permanent curly shaped hair> above, was wetted with tap water at 30°C for 30 seconds, and the wet tress was then wound around a plastic rod with a diameter of 14 mm, and fixed with a clip.
2. The rod was entirely covered with a plastic film for hermetical sealing. Thereafter, the tress was heated for 1 hour in an oven, which was set at 40°C.
3. The tress was removed from the oven, and cooled to a room temperature.
4. The tress was removed from the rod, and was then rinsed with running tap water at 30°C for 30 seconds. Thereafter, a shampoo for evaluation was lathered on the tress for 60 seconds.
5. The tress was rinsed with running tap water at 30°C for 30 seconds, and immersed at an infinite bath ratio in tap water at 30°C for 60 seconds. Thereafter, the tress was gently pulled up out of the water while the root thereof was held, and water was then drained off by lightly shaking it.
6. The tress was hung and allowed to stand in a laboratory for 2 hours, to be dried. The tress was combed, was then hung, and was then photographed from the side. Based on the photograph, the radius of curvature of the strongest curly portion in the tress was measured. The obtained value was doubled to obtain a curl diameter.

### (Evaluation criteria for the effect of giving shape to hair once more)

A: The curl diameter is 1 time or more and less than 2 times as large as the used rod (diameter: 14 mm)
B: The curl diameter is 2 times or more and less than 3 times as large as the used rod (diameter: 14 mm)
C: The curl diameter is 3 times or more and less than 4 times as large as the used rod (diameter: 14 mm)
D: The curl diameter is 4 times or more and less than 50 times as large as the used rod (diameter: 14 mm)
E: Straight hair was maintained, and the hair shape has not changed from before the treatment.

### <Formulation of shampoo for evaluation>

| Component | (mass%) |
|---|---|
| Sodium laureth sulfate | 15.5 |
| Lauramide DEA | 1.5 |
| Sodium benzoate | 0.5 |
| EDTA-2Na | 0.3 |
| Phosphoric acid | Amount for adjusting the pH to 7 |
| Deionized water | balance |
| Total | 100 |

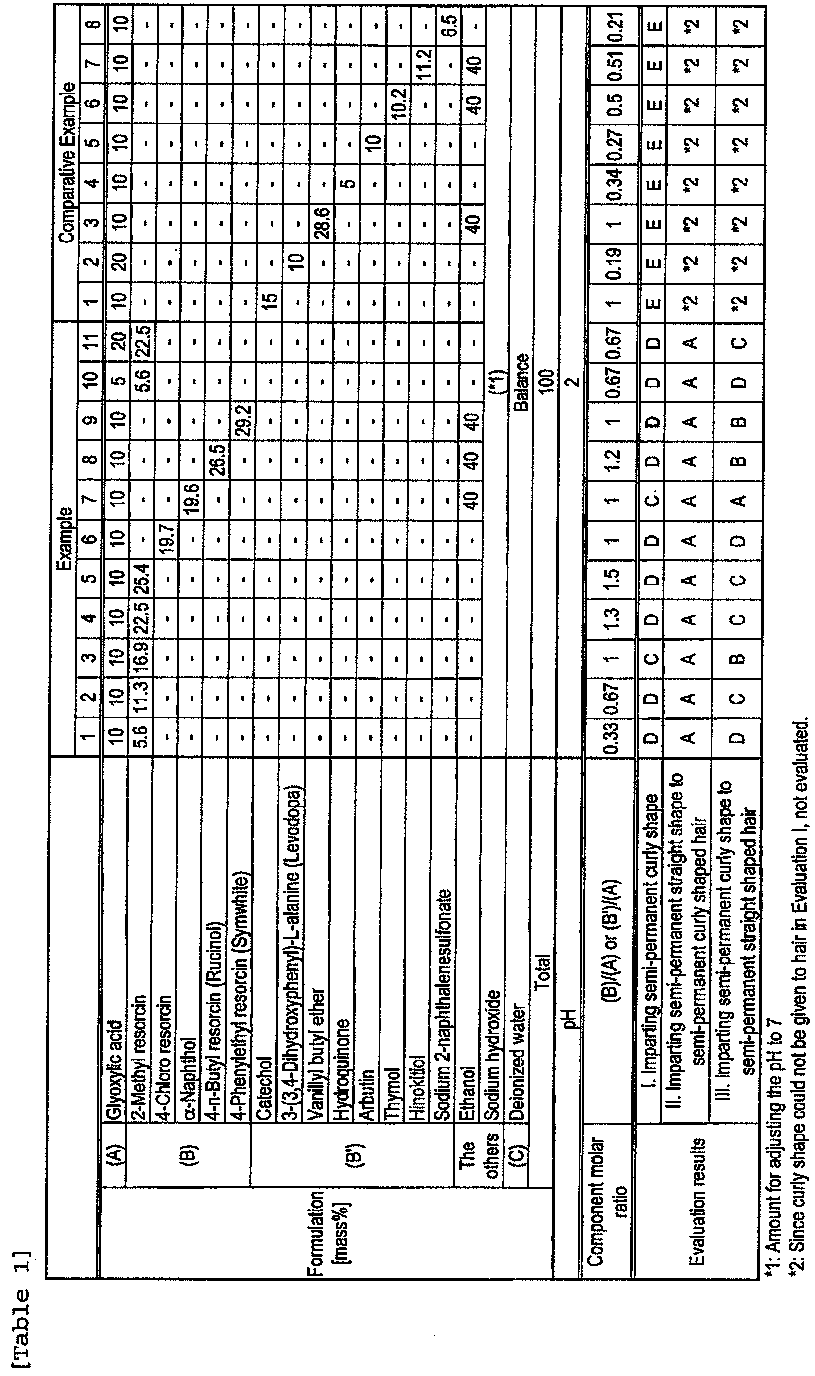

### Examples 12 and 14

The two-part treatment agents shown in Table 2, were prepared, and the following three-step hair treatment processes were then carried out using the agents. The shape-giving effect of each agent at each step was evaluated. The results are also shown in Table 2.

### <I: Imparting semi-permanent curly shape>

1. A tress in length of 25 cm, which consisted of 0.5 g of Caucasian straight hair (untreated hair), was wetted with tap water at 30°C for 30 seconds, and the wet tress was then wound around a plastic rod with a diameter of 14 mm, and fixed with a clip.
2. 1 g of the formulated first agent was applied to the tress wound around the rod, and the rod was entirely covered with a plastic film for hermetical sealing. The tress was then heated for 1 hour in an oven, which was set at 90°C.
3. The tress was removed from the oven, and cooled to a room temperature.
4. 1 g of the formulated second agent was applied to the tress wound around the rod. The rod was entirely covered with a plastic film for hermetical sealing, and it was then heated for 1 hour in an oven, which was set at 90°C.
5. The tress was removed from the oven, and cooled to a room temperature.
6. The tress was removed from the rod, and was then rinsed with running tap water at 30°C for 30 seconds. Thereafter, a shampoo for evaluation was lathered on the tress for 60 seconds.
7. The tress was rinsed with running tap water at 30°C for 30 seconds, and immersed at an infinite bath ratio in tap water at 30°C for 60 seconds. Thereafter, the tress was gently pulled up out of the water while the root thereof was held, and water was then drained off by lightly shaking it.
8. The tress was hung and allowed to stand in a laboratory for 2 hours, to be dried. The tress was combed, was then hung, and was then photographed from the side. Based on the photograph, the radius of curvature of the strongest curly portion in the tress was measured. The obtained value was doubled to obtain a curl diameter.

### (Evaluation criteria for the effect of giving initial shape to hair)

A: The curl diameter is 1 time or more and less than 2 times as large as the used rod (diameter: 14 mm)
B: The curl diameter is 2 times or more and less than 3 times as large as the used rod (diameter: 14 mm)
C: The curl diameter is 3 times or more and less than 4 times as large as the used rod (diameter: 14 mm)
D: The curl diameter is 4 times or more and less than 50 times as large as the used rod (diameter: 14 mm)
E: Straight hair was maintained, and the hair shape has not changed from before the treatment.

### <II: Imparting semi-permanent straight shape to semi-permanent curly shaped hair>

1. The tress, which had been evaluated in terms of <I: Imparting semi-permanent curly shape>, was combed to detangle it, and thereafter, a flat iron with an actual temperature of 180°C was slid through the tress at a rate of 5 cm/sec six times.
2. Tress was rinsed with running tap water at 30°C for 30 seconds, and a shampoo for evaluation was then lathered on the tress for 60 seconds. Thereafter, the tress was rinsed with running tap water at 30°C for 30 seconds, and was then dried with a towel.
3. The tress was dried, while being shaken, so that the natural shape of hair could appear (wherein dryer was not used), and it was then combed. The tress was hung, and was then visually observed from the side.

### (Evaluation criteria for the effect of giving shape to hair again)

A: The curl has not remained, and the hair is completely deformed into straight hair.
B: The curl has become weaker than before the treatment with a flat iron, but the hair has not been completely deformed into straight hair.
C: The curly hair remains as is, and the hair shape has not changed from before the treatment.

### <III: Imparting semi-permanent curly shape to semi-permanent straight shaped hair>

1. The tress, which had been evaluated in <II: Imparting semi-permanent straight shape to semi-permanent curly shaped hair>, was wetted with tap water at 30°C for 30 seconds, and the wet tress was then wound around a plastic rod with a diameter of 14 mm, and fixed with a clip.
2. The rod was entirely covered with a plastic film for hermetical sealing. Thereafter, the tress was heated for 1 hour in an oven, which was set at 40°C.
3. The tress was removed from the oven, and cooled to a room temperature.
4. The tress was removed from the rod, and was then rinsed with running tap water at 30°C for 30 seconds. Thereafter, a shampoo for evaluation was lathered on the tress for 60 seconds.
5. The tress was rinsed with running tap water at 30°C for 30 seconds, and immersed at an infinite bath ratio in tap water at 30°C for 60 seconds. Thereafter, the tress was gently pulled up out of the water while the root thereof was held, and water was then drained off by lightly shaking it.
6. The tress was hung and allowed to stand in a laboratory for 2 hours, to be dried. The tress was combed, was then hung, and was then photographed from the side. Based on the photograph, the radius of curvature of the strongest curly portion in the tress was measured. The obtained value was doubled to obtain a curl diameter.

### (Evaluation criteria for the effect of giving shape to hair once more)

A: The curl diameter is 1 time or more and less than 2 times as large as the used rod (diameter: 14 mm)
B: The curl diameter is 2 times or more and less than 3 times as large as the used rod (diameter: 14 mm)
C: The curl diameter is 3 times or more and less than 4 times as large as the used rod (diameter: 14 mm)
D: The curl diameter is 4 times or more and less than 50 times as large as the used rod (diameter: 14 mm)
E: Straight hair was maintained, and the hair shape has not changed from before the treatment.

**[Table 2]**

| | | | | Example | | |
|---|---|---|---|---|---|---|
| | | | | 12 | 13 | 14 |
| Formulated first agent | Formulation [mass%] | (B) | Resveratrol | 7.8 | - | - |
| | | | Phloretin | - | 9.3 | - |
| | | | 2,2'-4,4'-Tetrahydroxybenzophenone (benzophenone-2) | - | - | 8.4 |
| | | The others | Ethanol | 80 | 80 | 80 |
| | | | Sodium hydroxide | (*1) | | |
| | | (C) | Deionized water | Balance | | |
| | | Total | | 100 | | |
| | pH | | | 4.0 | | |
| Formulated second agent | Formulation [mass%] | (A) | Glyoxylic acid | 10 | 10 | 10 |
| | | The other | Sodium hydroxide | (*2) | | |
| | | (C) | Deionized water | Balance | | |
| | | Total | | 100 | | |
| | pH | | | 2.0 | | |
| Component molar ratio | | | (B)/(A) | 0.25 | 0.25 | 0.25 |
| Evaluation results | | | I. Imparting semi-permanent curly shape | A | B | C |
| | | | II. Imparting semi-permanent straight shape to semi-permanent curly shaped hair | A | A | A |
| | | | III. Imparting semi-permanent curly shape to semi-permanent straight shaped hair | B | C | C |

| | | | | | | |
|---|---|---|---|---|---|---|
| *1: Amount for adjusting the pH to 4.0 *2: Amount for adjusting the pH to 7 | | | | | | |

### Example 15

The treatment agent shown in Table 3 was prepared, and the following three-step hair treatment process was then carried out using the agent. The shape-giving effect of each agent at each treatment was evaluated. The results are also shown in Table 3.

### <I: Imparting semi-permanent straight shape>

1. A tress consisting of slightly spread, straight hair from Caucasian race (untreated hair) (weight: 0.5 g / length: 25 cm) was wetted with tap water at 30°C for 30 seconds, and the wet tress was then wound around a plastic rod with a diameter of 14 mm, and fixed with a clip.
2. 1.0 g of the treatment agent was applied to the tress wound around the rod, and the rod was entirely covered with a plastic film for hermetical sealing. Thereafter, it was heated for 1 hour in an oven, which was set at 40°C.
3. The tress was removed from the oven, and cooled to a room temperature.
4. The plastic film was uncovered, and the tress was then removed from the rod. Water was lightly removed with a towel, and the tress was then dried with hot air from a dryer, until it was completely dried.
5. The tress was combed to detangle it, and a flat iron with an actual temperature of 230°C was slid through the tress at a rate of 5 cm/sec six times, so that the style of straight hair was completely formed from the roots to the tips of the tress.
6. The tress was rinsed with running tap water at 30°C for 30 seconds. Thereafter, a shampoo for evaluation was lathered on the tress for 60 seconds. After that, the tress was rinsed with running tap water at 30°C for 30 seconds, and was then dried with a towel.
7. The tress was dried, while being shaken, so that the shape of hair could directly appear, and it was then combed. Thereafter, the tress was hung, and was then visually observed from the side.

### (Evaluation criteria)

A: The spreading of the tress disappears, and completely straight hair is maintained from the roots to the tips of the tress.
B: Although the tress is straight hair, in which the spreading of the tress is suppressed, it is slightly spread.
C: The spreading of the tress is equivalent to that of untreated hair.

### <II: Imparting semi-permanent curly shape to semi-permanent straight shaped hair>

1. The tress, which had been evaluated in <I: Imparting semi-permanent straight shape> above, was wetted with tap water at 30°C for 30 seconds, and the wet tress was then wound around a plastic rod with a diameter of 14 mm, and fixed with a clip.
2. The rod was entirely covered with a plastic film for hermetical sealing. Thereafter, the tress was heated for 1 hour in an oven, which was set at 40°C.
3. The tress was removed from the oven, and cooled to a room temperature.
4. The tress was removed from the rod, and was then rinsed with running tap water at 30°C for 30 seconds. Thereafter, a shampoo for evaluation was lathered on the tress for 60 seconds.
5. The tress was rinsed with running tap water at 30°C for 30 seconds, and immersed at an infinite bath ratio in tap water at 30°C for 60 seconds. Thereafter, the tress was gently pulled up out of the water while the root thereof was held, and water was then drained off by lightly shaking it.
6. The tress was hung and allowed to stand in a laboratory for 2 hours, to be dried. The tress was combed, was then hung, and was then photographed from the side. Based on the photograph, the radius of curvature of the strongest curly portion in the tress was measured. The obtained value was doubled to obtain a curl diameter.

### (Evaluation criteria)

A: The curl diameter is 1 time or more and less than 2 times as large as the used rod (diameter: 14 mm)
B: The curl diameter is 2 times or more and less than 3 times as large as the used rod (diameter: 14 mm)
C: The curl diameter is 3 times or more and less than 4 times as large as the used rod (diameter: 14 mm)
D: The curl diameter is 4 times or more and less than 50 times as large as the used rod (diameter: 14 mm)
E: Straight hair was maintained, and the hair shape has not changed from before the treatment.

### <III: Imparting semi-permanent straight shape to semi-permanent curly shaped hair>

1. The tress, which had been evaluated in <II: Imparting semi-permanent curly shape to semi-permanent straight shaped hair> above, was combed to detangle it, and a flat iron with an actual temperature of 180°C was then slid through the tress at a rate of 5 cm/sec six times.
2. The tress was rinsed with running tap water at 30°C for 30 seconds, and a shampoo for evaluation was then lathered on the tress for 60 seconds. Thereafter, the tress was rinsed with running tap water at 30°C for 30 seconds, and was then dried with a towel.
3. The tress was dried, while being shaken, so that the natural shape of hair could appear (wherein dryer was not used), and it was then combed. Thereafter, the tress was hung, and was then visually observed from the side.

### (Evaluation criteria)

A: The curl has not remained, and the hair has been completely deformed into straight hair
B: The curl has become weaker than before the treatment with a flat iron, but the hair has not been completely deformed into straight hair.
C: The curly hair remains as is, and the hair shape has not changed from before the treatment.

**[Table 3]**

| | | | Example |
|---|---|---|---|
| | | | 15 |
| Formulation [mass%] | (A) | Glyoxylic acid | 10 |
| | (B) | 2-Methyl resorcinol | 11.3 |
| | The other | Sodium hydroxide | (*1) |
| | (C) | Deionized water | Balance |
| | Total | | 100 |
| pH | | | 2.0 |
| Component molar ratio | | (B)/(A) | 0.67 |
| Evaluation results | | I. Imparting semi-permanent straight shape | A |
| | | II. Imparting semi-permanent curly shape to semi-permanent straight shaped hair | C |
| | | III. Imparting semi-permanent straight shape to semi-permanent curly shaped hair | A |

| | | | |
|---|---|---|---|
| *1: Amount for adjusting the pH to 7 | | | |

### Example 16 and 17

The treatment agents shown in Table 4 were prepared, and the following three-step hair treatment was then carried out using the agents. The effect of the agent to provide each hair shape was evaluated. The results are also shown in Table 4.

### <I: Imparting semi-permanent curly shape>

1. A tress in length of 25 cm, which consisted of 0.5 g of Caucasian straight hair, was wetted with tap water at 30°C for 30 seconds, and the wet tress was then wound around a plastic rod with a diameter of 14 mm, and fixed with a clip.
2. 1 g of the formulated first agent was applied to the tress wound around the rod. The rod was entirely covered with a plastic film for hermetical sealing, and it was then heated for 1 hour in an oven, which was set at 90°C.
3. The tress was removed from the oven, and cooled to a room temperature.
4. The tress was removed from the plastic film, and was then rinsed with running tap water at 30°C for 30 seconds. Thereafter, a shampoo for evaluation was lathered on the tress for 60 seconds, and the tress was then rinsed with running tap water at 30°C for 30 seconds.
5. The wet tress was then wound around a plastic rod with a diameter of 14 mm. Thereafter, it was then fixed with a clip.
6. 1 g of the formulated second agent was applied to the tress wound around the rod. The rod was entirely covered with a plastic film for hermetical sealing, and it was then heated for 1 hour in an oven, which was set at 90°C.
7. The tress was removed from the oven, and cooled to a room temperature.
8. The tress was removed from the rod, and was then rinsed with running tap water at 30°C for 30 seconds. Thereafter, a shampoo for evaluation was lathered on the tress for 60 seconds.
9. The tress was rinsed with running tap water at 30°C for 30 seconds, and immersed at an infinite bath ratio in tap water at 30°C for 60 seconds. Thereafter, the tress was gently pulled up out of the water while the root thereof was held, and water was then drained off by lightly shaking it.
10. The tress was hung and allowed to stand in a laboratory for 2 hours, to be dried. The tress was combed, was then hung, and was then photographed from the side. Based on the photograph, the radius of curvature of the strongest curly portion in the tress was measured. The obtained value was doubled to obtain a curl diameter.

The touch feeling of the tress of Examples 16 and 17 became much better than that in Examples 1 to 15.

### (Evaluation criteria)

A: The curl diameter is 1 time or more and less than 2 times as large as the used rod (diameter: 14 mm)
B: The curl diameter is 2 times or more and less than 3 times as large as the used rod (diameter: 14 mm)
C: The curl diameter is 3 times or more and less than 4 times as large as the used rod (diameter: 14 mm)
D: The curl diameter is 4 times or more and less than 50 times as large as the used rod (diameter: 14 mm)
E: Straight hair was maintained, and the hair shape has not changed from before the treatment.

### <II: Imparting semi-permanent straight shape to semi-permanent curly shaped hair>

1. The tress, which had been evaluated in <I: Imparting semi-permanent curly shape> above, was combed to detangle it, and a flat iron with an actual temperature of 180°C was then slid through the tress at a rate of 5 cm/sec six times.
2. The tress was rinsed with running tap water at 30°C for 30 seconds, and a shampoo for evaluation was then lathered on the tress for 60 seconds. Thereafter, the tress was rinsed with running tap water at 30°C for 30 seconds, and was then dried with a towel.
3. The tress was dried, while being shaken, so that the natural shape of hair could appear (wherein dryer was not used), and it was then combed. Thereafter, the tress was hung, and was then visually observed from the side.

### (Evaluation criteria)

A: The curl has not remained, and the hair is completely deformed into straight hair.
B: The curl has become weaker than before the treatment with a flat iron, but the hair has not been completely deformed into straight hair.
C: The curly hair remains as is, and the hair shape has not changed from before the treatment.

### <III: Imparting semi-permanent curly shape to semi-permanent straight shaped hair>

1. The tress, which had been evaluated in <II: Imparting semi-permanent straight shape to semi-permanent curly shaped hair> above, was wetted with tap water at 30°C for 30 seconds, and the wet tress was then wound around a plastic rod with a diameter of 14 mm, and fixed with a clip.
2. The rod was entirely covered with a plastic film for hermetical sealing. Thereafter, the tress was heated in an oven, which was set at 40°C, for 1 hour.
3. The tress was removed from the oven, and cooled to a room temperature.
4. The tress was removed from the rod, and was then rinsed with running tap water at 30°C for 30 seconds. Thereafter, a shampoo for evaluation was lathered on the tress for 60 seconds.
5. The tress was rinsed with running tap water at 30°C for 30 seconds, and immersed at an infinite bath ratio in tap water at 30°C for 60 seconds. Thereafter, the tress was gently pulled up out of the water while the root thereof was held, and water was then drained off by lightly shaking it.
6. The tress was hung and allowed to stand in a laboratory for 2 hours, to be dried. The tress was combed, was then hung, and was then photographed from the side. Based on the photograph, the radius of curvature of the strongest curly portion in the tress was measured. The obtained value was doubled to obtain a curl diameter.

### (Evaluation criteria)

A: The curl diameter is 1 time or more and less than 2 times as large as the used rod (diameter: 14 mm)
B: The curl diameter is 2 times or more and less than 3 times as large as the used rod (diameter: 14 mm)
C: The curl diameter is 3 times or more and less than 4 times as large as the used rod (diameter: 14 mm)
D: The curl diameter is 4 times or more and less than 50 times as large as the used rod (diameter: 14 mm)
E: Straight hair was maintained, and the hair shape has not changed from before the treatment.

**[Table 4]**

| | | | | Example | |
|---|---|---|---|---|---|
| | | | | 16 | 17 |
| Formulated first agent | Formulation [mass%] | (B) | 4-Phenylethyl resorcinol (Symwhite) | 10 | - |
| | | | Resveratrol | - | 10 |
| | | The others | Ethanol | 50 | 50 |
| | | | Sodium hydroxide | (*1) | |
| | | (C) | Deionized water | Balance | |
| | | Total | | 100 | |
| | pH | | | 4 | |
| Formulated second agent | Formulation [mass%] | (A) | Glyoxylic acid | 10 | 10 |
| | | The other | Sodium hydroxide | (*2) | |
| | | (C) | Deionized water | Balance | |
| | | Total | | 100 | |
| | pH | | | 2 | |
| Component molar ratio | | | (B)/(A) | 0.345 | 0.324 |
| Evaluation results | | | I. Imparting semi-permanent curly shape | A | B |
| | | | II. Imparting semi-permanent straight shape to semi-permanent curly shaped hair | A | A |
| | | | III. Imparting semi-permanent curly shape to semi-permanent straight shaped hair | B | C |

| | | | | | |
|---|---|---|---|---|---|
| *1: Amount for adjusting the pH to 4.0 *2: Amount for adjusting the pH to 7 | | | | | |

### Example 18

The following operations I and II are carried out.

### <I: Imparting semi-permanent curly shape>

A tress in length of 25 cm, which consists of 0.5 g of Caucasian straight hair (untreated hair), is wetted with tap water at 30°C for 30 seconds, and the wet tress is then wound around a plastic rod with a diameter of 14 mm, and fixed with a clip.

0.5 g of an aqueous solution of 20 mass% glyoxylic acid (pH = 2.0; adjusted by addition of sodium hydroxide) is applied to the tress wound around the rod, and it is then allowed to stand at 25°C for 5 minutes. Thereafter, 0.5 g of an aqueous solution of 30 mass% 4-phenylethyl resorcin (Symwhite) and 40 mass% ethanol (pH = 2.0; adjusted by addition of sodium hydroxide) is applied to the tress, and the rod is entirely covered with a plastic film for hermetical sealing. After that, the tress is heated for 1 hour in an oven, which is set at 90°C.

The tress is removed from the oven, and cooled to a room temperature. The tress is removed from the rod, and is then rinsed with running tap water at 30°C for 30 seconds. Thereafter, a shampoo for evaluation is lathered on the tress for 60 seconds. The tress is rinsed with running tap water at 30°C for 30 seconds, and immersed at an infinite bath ratio in tap water at 30°C for 60 seconds. Thereafter, the tress is gently pulled up out of the water while the root thereof is held, and water is then drained off by lightly shaking it. Thereafter, the tress is hung and allowed to stand in a laboratory for 2 hours, to be dried. Subsequently, the tress is combed, is then hung, and is then visually observed from the side. As a result, it is found that a semi-permanent curly shape is given to the tress.

### <II: Imparting semi-permanent straight shape to semi-permanent curly shaped hair>

The tress, which had been evaluated in <I: Imparting semi-permanent curly shape> above, is combed to detangle it, and a flat iron with an actual temperature of 180°C is slid through the tress at a rate of 5 cm/sec six times. Thereafter, the tress is rinsed with running tap water at 30°C for 30 seconds, and a shampoo for evaluation is then lathered on the tress for 60 seconds. Thereafter, the tress is rinsed with running tap water at 30°C for 30 seconds, and is then dried with a towel. The tress is dried, while being shaken, so that the natural shape of hair could appear, and it is then combed. Thereafter, the tress is hung, and is then visually observed from the side. As a result, it is found that a semi-permanent straight shape is given to the tress.

### Example 19

The following operations I and II are carried out.

### <I: Imparting semi-permanent curly shape>

A tress in length of 25 cm, which consisted of 0.5 g of Caucasian straight hair (untreated hair), is wetted with tap water at 30°C for 30 seconds, and the wet tress is then wound around a plastic rod with a diameter of 14 mm, and fixed with a clip.

0.5 g of an aqueous solution of 30 mass% 4-phenylethyl resorcin (Symwhite) and 40 mass% ethanol (pH = 2.0; adjusted by addition of sodium hydroxide) is applied to the tress wound around the rod, and it is then allowed to stand at 25°C for 5 minutes. Thereafter, 0.5 g of an aqueous solution of 20 mass% glyoxylic acid (pH = 2.0; adjusted by addition of sodium hydroxide) is applied to the tress, and the rod is entirely covered with a plastic film for hermetical sealing. After that, the tress is heated for 1 hour in an oven, which is set at 90°C.

The tress is removed from the oven, and cooled to a room temperature. The tress is removed from the rod, and is then rinsed with running tap water at 30°C for 30 seconds. Thereafter, a shampoo for evaluation is lathered on the tress for 60 seconds. After that, the tress is rinsed with running tap water at 30°C for 30 seconds, and immersed at an infinite bath ratio in tap water at 30°C for 60 seconds. Thereafter, the tress is gently pulled up out of the water while the root thereof is held, and water is then drained off by lightly shaking it. The tress is hung and allowed to stand in a laboratory for 2 hours, to be dried. Subsequently, the tress is combed, is then hung, and is then visually observed from the side. As a result, it is found that a semi-permanent curly shape is given to the tress.

### <II: Imparting semi-permanent straight shape to semi-permanent curly shaped hair>

The tress, which had been evaluated in <I: Imparting semi-permanent curly shape> above, is combed to detangle it, and a flat iron with an actual temperature of 180°C is slid through the tress at a rate of 5 cm/sec six times. Thereafter, the tress is rinsed with running tap water at 30°C for 30 seconds, and a shampoo for evaluation is then lathered on the tress for 60 seconds. After that, the tress is rinsed with running tap water at 30°C for 30 seconds, and is then dried with a towel. The tress is dried, while being shaken, so that the natural shape of hair could appear, and it is then combed. Thereafter, the tress is hung, and is then visually observed from the side. As a result, it is found that a semi-permanent straight shape is given to the tress.

### Example 20

The following operations STEPS 1 to 3 are carried out.

### <STEP 1: Imparting semi-permanent straight shape>

An aqueous solution of 20 mass% glyoxylic acid (pH = 2.0; adjusted by addition of sodium hydroxide) is mixed with an aqueous solution of 30 mass% 4-phenylethyl resorcin (Symwhite) and 40 mass% ethanol (pH = 2.0; adjusted by addition of sodium hydroxide) at a mixing ratio of 1 : 1, to prepare a treatment agent A. A tress in length of 25 cm, which consisted of 0.5 g of Caucasian curly hair, is wetted with tap water at 30°C for 30 seconds, and 1 g of the treatment agent A is then applied to the wet tress. Thereafter, the tress is entirely covered with a plastic film for hermetical sealing, and it is then heated for 1 hour in an oven, which is set at 40°C.

The tress is removed from the oven, and cooled to a room temperature. The plastic film is removed, and an excessive treatment agent is removed from the hair with a towel. Thereafter, the hair is dried with a dryer, while being combed.

After completion of the drying with a dryer, using a flat iron with a preset temperature of 230°C, the root side of the tress is held with the plates of the flat iron, and an operation to slide the flat iron from the roots to the tips of the tress at a rate of 5 cm/sec, while the hair is held with the plates, is carried out six times.

After completion of the iron treatment, the tress is rinsed with running tap water at 30°C for 30 seconds, and a shampoo for evaluation is then lathered on the tress for 60 seconds. Thereafter, the tress is rinsed with running tap water at 30°C for 30 seconds, is then dried with a towel, and is then naturally dried. As a result, a semi-permanent straight shape is given to the resulting tress.

### <STEP 2: Imparting semi-permanent curly shape>

The tress, which had been evaluated in STEP 1 above, is wetted with tap water at 30°C for 30 seconds, and the wet tress is then wound around a plastic rod with a diameter of 14mm, and fixed with a clip. The rod is entirely covered with a plastic film for hermetical sealing, and the tress is then heated for 1 hour in an oven, which is set at 40°C. Thereafter the tress is removed from the oven, and cooled to a room temperature. The tress is removed from the rod, and is then rinsed with running tap water at 30°C for 30 seconds. Thereafter, a shampoo for evaluation is then lathered on the tress for 60 seconds. The tress is rinsed with running tap water at 30°C for 30 seconds, and immersed at an infinite bath ratio in tap water at 30°C for 60 seconds. Thereafter, the tress is gently pulled up out of the water while the root of the tress is held, and water is then drained off by lightly shaking it. The tress is hung and allowed to stand in a laboratory for 2 hours, to be dried. The tress is combed, is then hung, and is then visually observed from the side. As a result, it is found that a semi-permanent curly shape is given to the tress.

### <STEP 3: Imparting semi-permanent straight shape>

The tress, which had been evaluated in STEP 2 above, is combed to detangle it, and a flat iron with an actual temperature of 180°C is slid through the tress at a rate of 5 cm/sec six times. The tress is rinsed with running tap water at 30°C for 30 seconds, and a shampoo for evaluation is then lathered on the tress for 60 seconds. Thereafter, the tress is rinsed with running tap water at 30°C for 30 seconds, and is then dried with a towel. The tress is dried, while being shaken, so that the natural shape of hair could appear, and it is then combed. Thereafter, the tress is hung, and is then visually observed from the side. As a result, it is found that a semi-permanent straight shape is given to the tress.

## Claims

1. An agent for hair deforming treatment comprising the following components (A), (B) and (C):
(A) glyoxylic acid, or a hydrate or a salt thereof,
(B) a compound represented by the following formula (1), which has a molecular weight of more than 120, or a compound represented by the following formula (1-3-a) or (1-3-b) and
(C) water: 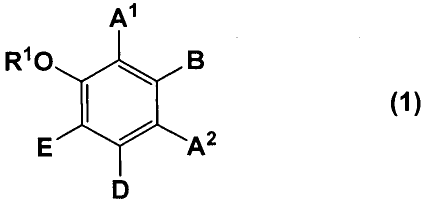 wherein, in the formula (1),
R¹ represents a hydrogen atom or a methyl group,
A¹ and A², which are optionally the same or different, each represent a hydrogen atom, a straight-chain or branched-chain alkyl group or alkenyl group containing from 1 to 12 carbon atoms, an optionally substituted aralkyl group or arylalkenyl group containing from 7 to 12 carbon atoms, a straight-chain or branched-chain alkoxy group or alkenyloxy group containing from 1 to 6 carbon atoms, a halogen atom, or -CO-R² (wherein R² represents a straight-chain or branched-chain alkyl group or alkenyl group containing from 1 to 12 carbon atoms, an optionally substituted aralkyl group or arylalkenyl group containing from 7 to 12 carbon atoms, or an optionally substituted aromatic hydrocarbon group containing from 6 to 12 carbon atoms),
B represents a hydrogen atom, a straight-chain or branched-chain alkyl group or alkenyl group containing from 1 to 12 carbon atoms, an optionally substituted aralkyl group or arylalkenyl group containing from 7 to 12 carbon atoms, - OR³, or -COOR³ (wherein R³ represents a hydrogen atom, or a straight-chain or branched-chain alkyl group or alkenyl group containing from 1 to 6 carbon atoms),
D represents a hydroxy group or a straight-chain or branched-chain alkoxy group or alkenyloxy group containing from 1 to 12 carbon atoms, and
E represents a hydrogen atom, a hydroxy group, a straight-chain or branched-chain alkyl group or alkenyl group containing from 1 to 6 carbon atoms, or a straight-chain or branched-chain alkoxy group or alkenyloxy group containing from 1 to 6 carbon atoms,
provided that two or three of A¹, A², B and E are hydrogen atoms, and the remaining groups do not include sulfo groups; 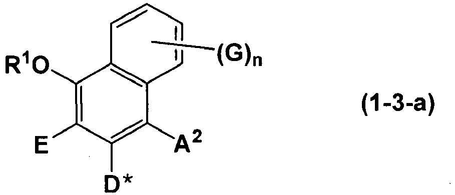 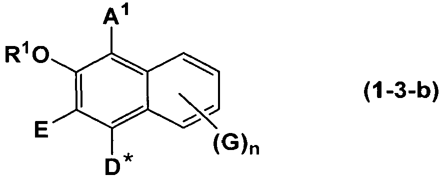
wherein, in the formulae (1-3-a) and (1-3-b),
R¹, A¹, A² and E are as defined above;
D* represents a hydrogen atom, hydroxy group, a methyl group or a straight-chain or branched-chain alkoxy group or alkenyloxy group containing from 1 to 12 carbon atoms;
G represents a hydroxy group, a straight-chain or branched-chain alkyl group or alkenyl group containing from 1 to 6 carbon atoms, or an alkoxy group containing from 1 to 6 carbon atoms, and
n represents an integer of from 0 to 2.

2. The hair deforming treatment agent according to claim 1, wherein the molar ratio of the content of the component (B) to the content of the component (A), (B)/(A), is 0.1 or more and 5 or less.

3. The hair deforming treatment agent according to claim 1 or 2, wherein the component (B) is a compound represented by the following formula (1-1-3): 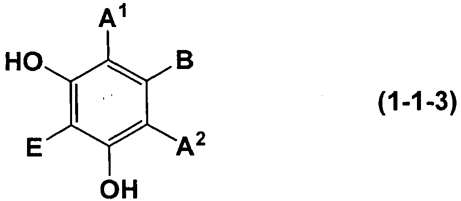 wherein A¹, A², B and E are as defined above.

4. The hair deforming treatment agent according to any one of claims 1 to 3, wherein the component (B) is one or two or more selected from the group consisting of 2-alkyl resorcin, 4-alkyl resorcin, 4-aralkyl resorcin, and 4-halogenated resorcin.

5. The hair deforming treatment agent according to any one of claims 1 to 4, wherein the content of the component (A) is, in terms of glyoxylic acid, 1 mass% or more and 30 mass% or less.

6. The hair deforming treatment agent according to any one of claims 1 to 5, wherein the content of the component (B) is 0.2 mass% or more and 30 mass% or less.

7. The hair deforming treatment agent according to any one of claims 1 to 6, wherein the pH thereof is 4 or less.

8. The hair deforming treatment agent according to any one of claims 1 to 7, wherein the total amount of thiol, hydrogen sulfite and salts thereof is less than 0.1 mass%.

9. The hair deforming treatment agent according to any one of claims 1 to 8, which is a multi-part agent comprising a first agent comprising the component (B) and a second agent comprising the component (A), wherein the pH of the second agent is 4 or less.

10. A method of hair treatment for semi-permanently or permanently deforming the shape of hair, which comprises the following steps (i) and (ii):
(i) applying the hair deforming treatment agent according to any one of claims 1 to 9 to hair and then allowing the agent to penetrate into the hair, and
(ii) heating and shaping the hair into which the hair deforming treatment agent has penetrated.

11. The method of hair treatment according to claim 10, wherein the step (i) is a step of applying the first agent in the hair deforming treatment agent according to claim 9 to hair, and then applying the second agent onto the portion applied.

12. The method of hair treatment according to claim 11, which does not comprise a step of rinsing the first agent after application of the first agent to hair and before application of the second agent, wherein the molecular weight of the component (B) comprised in the first agent is from 100 to 180.

13. The method of hair treatment according to claim 11, which comprises a step of rinsing the first agent after application of the first agent to hair and before application of the second agent, wherein the molecular weight of the component (B) comprised in the first agent is from 140 to 1,000.

14. The method of hair treatment according to any one of claims 10 to 13, which comprises a step of wetting hair before the step (i).

15. The method of hair treatment according to any one of claims 10 to 14, wherein the heating temperature in the step (ii) is 50°C or higher and 250°C or lower.

16. The method of hair treatment according to any one of claims 10 to 15, wherein the step (ii) is carried out under an environment in which evaporation of water is suppressed.

17. The method of hair treatment according to any one of claims 10 to 16, which does not comprise a step of applying a hair treatment agent comprising a reducing agent or a strongly-alkaline hair treatment agent having pH of from 12 to 14 to the hair.

18. The method of hair treatment according to any one of claims 10 to 17, wherein the hair is re-deformed into a different shape by heating after the step (ii).

## Patentansprüche

1. Mittel zur Haardeformationsbehandlung, enthaltend die folgenden Komponenten (A), (B) und (C):
(A) Glyoxalsäure oder ein Hydrat oder ein Salz davon,
(B) eine Verbindung mit der folgenden Formel (1), die ein Molekulargewicht von mehr als 120 hat, oder eine Verbindung mit der folgenden Formel (1-3-a) oder (1-3-b) und
(C) Wasser: 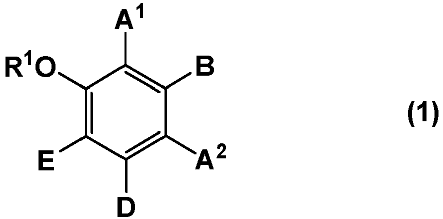 worin in der Formel (1)
R¹ ein Wasserstoffatom oder eine Methylgruppe ist,
A¹ und A², die wahlweise gleich oder verschieden sind, jeweils ein Wasserstoffatom, eine geradkettige oder verzweigte Alkylgruppe oder Alkenylgruppe mit 1 bis 12 Kohlenstoffatomen, eine wahlweise substituierte Aralkylgruppe oder Arylalkenylgruppe mit 7 bis 12 Kohlenstoffatomen, geradkettige oder verzweigte Alkoxygruppe oder Alkenyloxygruppe mit 1 bis 6 Kohlenstoffatomen, Halogenatom oder -CO-R² sind (worin R² eine geradkettige oder verzweigte Alkylgruppe oder Alkenylgruppe mit 1 bis 12 Kohlenstoffatomen, wahlweise substituierte Aralkylgruppe oder Arylalkenylgruppe mit 7 bis 12 Kohlenstoffatomen oder wahlweise substituierte aromatische Kohlenwasserstoffgruppe mit 6 bis 12 Kohlenstoffatomen ist),
B ein Wasserstoffatom, geradkettige oder verzweigte Alkylgruppe oder Alkenylgruppe mit 1 bis 12 Kohlenstoffatomen, wahlweise substituierte Aralkylgruppe oder Arylalkenylgruppe mit 7 bis 12 Kohlenstoffatomen, OR³ oder -COOR³ ist (worin R³ ein Wasserstoffatom oder eine geradkettige oder verzweigte Alkylgruppe oder Alkenylgruppe mit 1 bis 6 Kohlenstoffatomen ist),
D eine Hydroxygruppe oder geradkettige oder verzweigte Alkoxygruppe oder Alkenyloxygruppe mit 1 bis 12 Kohlenstoffatomen ist und
E ein Wasserstoffatom, Hydroxygruppe, geradkettige oder verzweigte Alkylgruppe oder Alkenylgruppe mit 1 bis 6 Kohlenstoffatomen oder eine geradkettige oder verzweigte Alkoxygruppe oder Alkenyloxygruppe mit 1 bis 6 Kohlenstoffatomen ist,
vorausgesetzt, dass zwei oder drei von A¹, A², B und E Wasserstoffatome sind und die verbleibenden Gruppen keine Sulfogruppen enthalten, 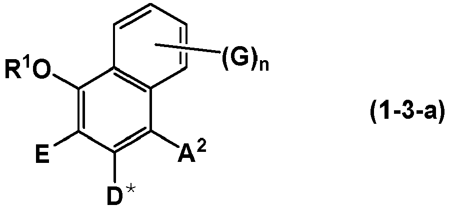 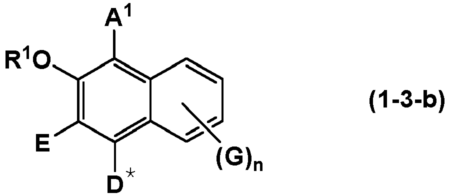
worin in den Formeln (1-3-a) und (1-3-b) R¹, A¹, A² und E wie oben definiert sind,
D* ein Wasserstoffatom, Hydroxygruppe, Methylgruppe oder geradkettige oder verzweigte Alkoxygruppe oder Alkenyloxygruppe mit 1 bis 12 Kohlenstoffatomen ist,
G eine Hydroxygruppe, geradkettige oder verzweigte Alkylgruppe oder Alkenylgruppe mit 1 bis 6 Kohlenstoffatomen oder Alkoxygruppe mit 1 bis 6 Kohlenstoffatomen ist und
n eine ganze Zahl von 0 bis 2 ist.

2. Haardeformationsbehandlungsmittel gemäß Anspruch 1, worin das molare Verhältnis des Gehaltes der Komponente (B) zu dem Gehalt der Komponente (A), (B)/(A), 0,1 oder mehr und 5 oder weniger ist.

3. Haardeformationsbehandlungsmittel gemäß Anspruch 1 oder 2, worin die Komponente (B) eine Verbindung mit der folgenden Formel (1-1-3) ist: 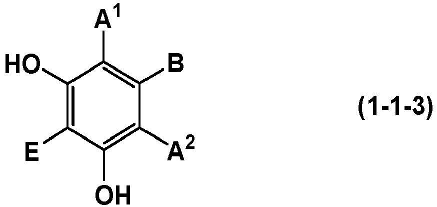 worin A¹, A², B und E wie oben definiert sind.

4. Haardeformationsbehandlungsmittel gemäß einem der Ansprüche 1 bis 3, worin die Komponente (B) eine oder zwei oder mehrere ist, ausgewählt aus der Gruppe, bestehend aus 2-Alkylresorcin, 4-Alkylresorcin, 4-Aralkylresorcin und 4-halogeniertem Resorcin.

5. Haardeformationsbehandlungsmittel gemäß einem der Ansprüche 1 bis 4, worin der Gehalt der Komponente (A), ausgedrückt als Glyoxalsäure, 1 Masse-% oder mehr und 30 Masse-% oder weniger ist.

6. Haardeformationsbehandlungsmittel gemäß einem der Ansprüche 1 bis 5, worin der Gehalt der Komponente (B) 0,2 Masse-% oder mehr und 30 Masse-% oder weniger ist.

7. Haardeformationsbehandlungsmittel gemäß einem der Ansprüche 1 bis 6, worin der pH davon 4 oder weniger ist.

8. Haardeformationsbehandlungsmittel gemäß einem der Ansprüche 1 bis 7, worin die Gesamtmenge an Thiol, Hydrogensulfit und Salzen davon weniger als 0,1 Masse-% ist.

9. Haardeformationsbehandlungsmittel gemäß einem der Ansprüche 1 bis 8, die ein Multiteilmittel ist, enthaltend ein erstes Mittel, das die Komponente (B) enthält, und ein zweites Mittel, das die Komponente (A) enthält, worin der pH des zweiten Mittels 4 oder weniger ist.

10. Verfahren zur Haarbehandlung für semi-permanentes oder permanentes Deformieren der Form von Haar, enthaltend die folgenden Schritte (i) und (ii):
(i) Auftragen des Haardeformationsbehandlungsmittel gemäß einem der Ansprüche 1 bis 9 auf Haar und anschließendes Ermöglichen, dass das Mittel in das Haar eindringen kann, und
(ii) Erwärmen und Formen des Haars, in das das Haardeformationsbehandlungsmittel eingedrungen ist.

11. Verfahren zur Haarbehandlung gemäß Anspruch 10, worin der Schritt (i) ein Schritt zum Auftragen des ersten Mittels in dem Haardeformationsbehandlungsmittel gemäß Anspruch 9 auf das Haar und das anschließende Auftragen des zweiten Mittels auf den aufgetragenen Bereich ist.

12. Verfahren zur Haarbehandlung gemäß Anspruch 11, das keinen Schritt zum Spülen des ersten Mittels nach Auftragung des ersten Mittels auf das Haar und vor Auftragung des zweiten Mittels enthält, worin das Molekulargewicht der Komponente (B), die im ersten Mittel enthalten ist, von 100 bis 180 ist.

13. Verfahren zur Haarbehandlung gemäß Anspruch 11, enthaltend einen Schritt zum Spülen des ersten Mittels nach Auftragung des ersten Mittels auf das Haar und vor Auftragung des zweiten Mittels, worin das Molekulargewicht der Komponente (B), die im ersten Mittel enthalten ist, von 140 bis 1.000 ist.

14. Verfahren zur Haarbehandlung gemäß einem der Ansprüche 10 bis 13, enthaltend einen Schritt zum Benetzen des Haars vor dem Schritt (i).

15. Verfahren zur Haarbehandlung gemäß einem der Ansprüche 10 bis 14, worin die Erwärmungstemperatur im Schritt (ii) 50°C oder mehr und 250°C oder weniger ist.

16. Verfahren zur Haarbehandlung gemäß einem der Ansprüche 10 bis 15, worin der Schritt (ii) in einer Umgebung durchgeführt wird, bei der die Verdampfung von Wasser unterdrückt wird.

17. Verfahren zur Haarbehandlung gemäß einem der Ansprüche 10 bis 16, das keinen Schritt zum Auftragen eines Haarbehandlungsmittels, das ein Reduktionsmittel enthält, oder eines stark alkalischen Haarbehandlungsmittels mit einem pH von 12 bis 14 auf das Haar enthält.

18. Verfahren zur Haarbehandlung gemäß einem der Ansprüche 10 bis 17, worin das Haar zu einer anderen Form durch Erwärmen nach dem Schritt (ii) erneut deformiert wird.

## Revendications

1. Agent de traitement de déformation des cheveux comprenant les composants (A), (B) et (C) suivants :
(A) acide glyoxylique, ou un hydrate ou un sel de celui-ci,
(B) un composé représenté par la formule (1) suivante, qui présente un poids moléculaire de plus de 120, ou un composé représenté par la formule (1-3-a) ou (1-3-b) suivante et
(C) eau : 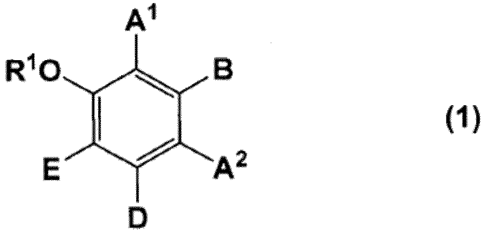 dans lequel, dans la formule (1),
R¹ représente un atome d'hydrogène ou un groupe méthyle,
A¹ et A², qui sont facultativement identiques ou différents, représentent chacun un atome d'hydrogène, un groupe alkyle ou un groupe alcényle à chaîne linéaire ou à chaîne ramifiée contenant de 1 à 12 atomes de carbone, un groupe aralkyle ou un groupe arylalcényle facultativement substitué contenant de 7 à 12 atomes de carbone, un groupe alcoxy ou un groupe alcényloxy à chaîne linéaire ou à chaîne ramifiée contenant de 1 à 6 atomes de carbone, un atome d'halogène ou -CO-R² (dans lequel R² représente un groupe alkyle ou un groupe alcényle à chaîne linéaire ou à chaîne ramifiée contenant de 1 à 12 atomes de carbone, un groupe aralkyle ou un groupe arylalcényle facultativement substitué contenant de 7 à 12 atomes de carbone ou un groupe hydrocarboné aromatique facultativement substitué contenant de 6 à 12 atomes de carbone),
B représente un atome d'hydrogène, un groupe alkyle ou un groupe alcényle à chaîne linéaire ou à chaîne ramifiée contenant de 1 à 12 atomes de carbone, un groupe aralkyle ou un groupe arylalcényle facultativement substitué contenant de 7 à 12 atomes de carbone, -OR³ ou -COOR³ (dans lequel R³ représente un atome d'hydrogène ou un groupe alkyle ou un groupe alcényle à chaîne linéaire ou à chaîne ramifiée contenant de 1 à 6 atomes de carbone),
D représente un groupe hydroxy ou un groupe alcoxy ou un groupe alcényloxy à chaîne linéaire ou à chaîne ramifiée contenant de 1 à 12 atomes de carbone, et
E représente un atome d'hydrogène, un groupe hydroxy, un groupe alkyle ou un groupe alcényle à chaîne linéaire ou à chaîne ramifiée contenant de 1 à 6 atomes de carbone, ou un groupe alcoxy ou un groupe alcényloxy à chaîne linéaire ou à chaîne ramifiée contenant de 1 à 6 atomes de carbone,
à condition que deux ou trois parmi A¹, A², B et E soient des atomes d'hydrogène, et que les groupes restants n'incluent pas de groupes sulfo ; 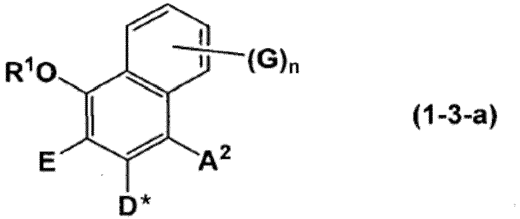 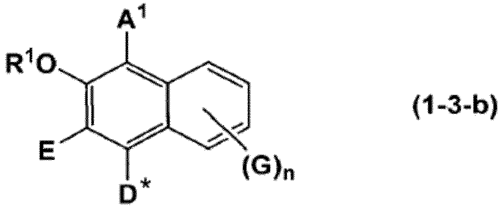
dans lequel, dans les formules (1-3-a) et (1-3-b),
R¹, A¹, A² et E sont tels que définis ci-dessus ;
D* représente un atome d'hydrogène, un groupe hydroxy, un groupe méthyle ou un groupe alcoxy ou un groupe alcényloxy à chaîne linéaire ou à chaîne ramifiée contenant de 1 à 12 atomes de carbone ;
G représente un groupe hydroxy, un groupe alkyle ou un groupe alcényle à chaîne linéaire ou à chaîne ramifiée contenant de 1 à 6 atomes de carbone, ou un groupe alcoxy contenant de 1 à 6 atomes de carbone, et
n représente un nombre entier de 0 à 2.

2. Agent de traitement de déformation des cheveux selon la revendication 1, dans lequel le rapport molaire de la teneur en composant (B) sur la teneur en composant (A), (B)/(A), est 0,1 ou plus et 5 ou moins.

3. Agent de traitement de déformation des cheveux selon la revendication 1 ou 2, dans lequel le composant (B) est un composé représenté par la formule (1-1-3) suivante : 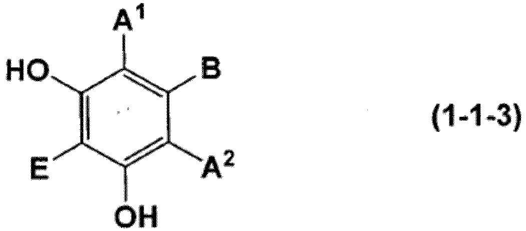 dans lequel A¹, A², B et E sont tels que définis ci-dessus.

4. Agent de traitement de déformation des cheveux selon l'une quelconque des revendications 1 à 3, dans lequel le composant (B) est un ou deux ou plus choisis dans le groupe consistant en la 2-alkyl-résorcine, la 4-alkyl-résorcine, la 4-arakyl-résorcine et la 4-halogéno-résorcine.

5. Agent de traitement de déformation des cheveux selon l'une quelconque des revendications 1 à 4, dans lequel la teneur en composant (A) est, en termes d'acide glyoxylique, de 1 % en masse ou plus et de 30 % en masse ou moins.

6. Agent de traitement de déformation des cheveux selon l'une quelconque des revendications 1 à 5, dans lequel la teneur en composant (B) est de 0,2 % en masse ou plus et de 30 % en masse ou moins.

7. Agent de traitement de déformation des cheveux selon l'une quelconque des revendications 1 à 6, dans lequel le pH de celui-ci est de 4 ou moins.

8. Agent de traitement de déformation des cheveux selon l'une quelconque des revendications 1 à 7, dans lequel la quantité totale de thiol, d'hydrogénosulfite et de sels de ceux-ci est inférieure à 0,1 % en masse.

9. Agent de traitement de déformation des cheveux selon l'une quelconque des revendications 1 à 8, qui est un agent en plusieurs parties comprenant un premier agent comprenant le composant (B) et un second agent comprenant le composant (A), dans lequel le pH du second agent est de 4 ou moins.

10. Procédé de traitement capillaire pour déformer de façon semi-permanente ou permanente la forme des cheveux, qui comprend les étapes (i) et (ii) suivantes :
(i) appliquer l'agent de traitement de déformation des cheveux selon l'une quelconque des revendications 1 à 9 sur les cheveux et ensuite laisser l'agent pénétrer à l'intérieur des cheveux, et
(ii) chauffer et mettre en forme les cheveux à l'intérieur desquels l'agent de traitement de déformation des cheveux a pénétré.

11. Procédé de traitement capillaire selon la revendication 10, dans lequel l'étape (i) est une étape d'application du premier agent dans l'agent de traitement de déformation des cheveux selon la revendication 9 sur les cheveux, et ensuite d'application du second agent sur la partie appliquée.

12. Procédé de traitement capillaire selon la revendication 11, qui ne comprend pas une étape de rinçage du premier agent après l'application du premier agent sur les cheveux et avant l'application du second agent, dans lequel le poids moléculaire du composant (B) compris dans le premier agent est de 100 à 180.

13. Procédé de traitement capillaire selon la revendication 11, qui comprend une étape de rinçage du premier agent après l'application du premier agent sur les cheveux et avant l'application du second agent, dans lequel le poids moléculaire du composant (B) compris dans le premier agent est de 140 à 1 000.

14. Procédé de traitement capillaire selon l'une quelconque des revendications 10 à 13, qui comprend une étape de mouillage des cheveux avant l'étape (i).

15. Procédé de traitement capillaire selon l'une quelconque des revendications 10 à 14, dans lequel la température de chauffage dans l'étape (ii) est de 50 °C ou plus et de 250 °C ou moins.

16. Procédé de traitement capillaire selon l'une quelconque des revendications 10 à 15, dans lequel l'étape (ii) est effectuée dans un environnement dans lequel l'évaporation de l'eau est supprimée.

17. Procédé de traitement capillaire selon l'une quelconque des revendications 10 à 16, qui ne comprend pas une étape d'application d'un agent de traitement capillaire comprenant un agent réducteur ou un agent de traitement capillaire fortement alcalin présentant un pH de 12 à 14 sur les cheveux.

18. Procédé de traitement capillaire selon l'une quelconque des revendications 10 à 17, dans lequel les cheveux sont redéformés en une forme différente par chauffage après l'étape (ii).
